# EUROPEAN PATENT SPECIFICATION

(11) **EP 4 587 841 B1**
(45) Date of publication and mention of the grant of the patent: **19.08.2026**
(21) Application number: 23765531.1
(22) Date of filing: 12.09.2023
(51) Int. Cl.: G01N 33/68, G01N 33/557

(54) **METHODS TO DETERMINE DRUG TARGET RESIDENCE TIME AND TO SELECT BEST DRUG-TARGET CANDIDATES**
VERFAHREN ZUR BESTIMMUNG DER ARZNEIMITTELZIELVERWEILZEIT UND ZUR AUSWAHL DER BESTEN ARZNEIMITTELZIELKANDIDATEN
PROCÉDÉS POUR DÉTERMINER LE TEMPS DE SÉJOUR D'UNE CIBLE DE MÉDICAMENT ET POUR SÉLECTIONNER LES MEILLEURS CANDIDATS DE CIBLE DE MÉDICAMENT

(30) Priority: 14.09.2022 EP 22195525
(43) Date of publication of application: 23.07.2025
(73) Proprietor: Biognosys AG, 8952 Schlieren (CH)
(72) Inventor: SABINO, Fabio Mira Rocha, 2850 Nærum (DK); PECNIK, Jaruschka Aisha, 8037 Zurich (CH); BRUDERER, Roland, 8406 Winterthur (CH)
(74) Representative: Bremi, Tobias Hans
(86) International application number: PCT/EP2023/074951
(87) International publication number: WO 2024/056627

(56) References cited:
- WO-A1-2014/082733
- SCHUETZ DORIS A. ET AL: "Kinetics for Drug Discovery: an industry-driven effort to target drug residence time", DRUG DISCOVERY TODAY, vol. 22, no. 6, 1 June 2017 (2017-06-01), AMSTERDAM, NL, pages 896 - 911, XP093020509, ISSN: 1359-6446, DOI: 10.1016/j.drudis.2017.02.002
- SCHOPPER SIMONE ET AL: "Measuring protein structural changes on a proteome-wide scale using limited proteolysis-coupled mass spectrometry", vol. 12, no. 11, 26 October 2017 (2017-10-26), GB, pages 2391 - 2410, XP055922516, ISSN: 1754-2189, Retrieved from the Internet <URL:http://www.nature.com/articles/nprot.2017.100> DOI: 10.1038/nprot.2017.100

## Description

### TECHNICAL FIELD

The present invention relates to methods for the determination of the residence time between at least one Target and at least one Ligand, optionally in their native biological context, using limited proteolysis e.g., combined with selected reaction monitoring, parallel reaction monitoring, data-independent acquisition (DIA), including Sequential Windowed Acquisition of All Theoretical Fragment Ion Mass Spectra (SWATH) methods and the like.

### PRIOR ART

Proteins are crucial effectors and regulators of a wide variety of cellular processes. In response to perturbations (for example, in case of disease), they can change their cellular concentration and their structure. Being able to capture such transitions is an essential task in life sciences, to understand the functioning of basic cellular processes in health and disease and to identify new options for disease diagnosis and treatment. Changes in cellular protein concentration in response to perturbations can be routinely probed by mass spectrometry (MS) based-proteomic techniques. Much less is known about switches in cellular protein conformation, mostly due to the lack of suitable approaches to study protein folds in cells. This is a substantial limitation for biological and clinical applications, since conformational changes can strongly impact protein activity, thus profoundly affecting a cell's physiology.

Proteins can change their conformation upon binding to lipids, ions, small molecules or nucleic acids, interaction with other proteins, chemical modification (e.g., phosphorylation) or environmental changes, such as varying pH or temperature. The extent of a conformational change ranges from small local motions, such as allosteric rearrangements, through larger scale fluctuations, such as domain motions, to the drastic switch between folded and unfolded or monomeric and polymeric states. In particular, the transition of monomeric proteins to higher order aggregated structures has gained increasing attention recently, in both biology and biomedicine. Over the last two decades, a variety of human diseases (more than 20 different pathologies), referred to as protein aggregation diseases were shown to be associated with the intracellular or extracellular accumulation of aggregates of specific misfolded proteins. Many neurodegenerative diseases, such as Parkinson's disease or Alzheimer's disease, of previously unknown aetiology now fall into this category. The different diseases can even be classified according to the major protein components of their aggregates, which also distinguish their clinical manifestations. For example, αSynuclein (αSyn)-containing Lewy bodies are typical for most types of Parkinsonism (PD), while amyloid-^{β} peptide inclusions are produced in Alzheimer's disease (see. e.g. A Aguzzi & T O'Connor, Nat Rev Drug Discov 9 (3), 237). The possibility of monitoring such protein conformational transitions in biological specimens would open new possibilities for the diagnosis and therapy of these protein-centric conditions and shed light on their pathogenesis.

A number of biophysical techniques have been applied to monitor conformational features of proteins, such as nuclear magnetic resonance (NMR), X-ray crystallography, infrared and Raman spectroscopy, circular dichroism, atomic force microscopy or fluorescence spectroscopy. These techniques are predominantly used to analyze (purified) proteins in vitro, due to their incapability of dealing with complex biological backgrounds. This is a substantial limitation, since the conformation adopted by a protein is regulated in cells by multiple co-occurring events specific to its cellular context, such as environmental cues, binding events or post translational modifications, which cannot be recapitulated by in vitro systems. Techniques based on Förster resonance energy transfer (FRET) offer the advantage of monitoring conformational changes of proteins in their native cellular environment, but require the introduction of fluorescent probes at suitable sites of each Target protein and are not applicable on a large scale or on clinical samples.

Gupta, Lapadula and Abou-Donia in a paper entitled "Purification and Characterization of Cytochrome P450 Isozymes from β-Naphthoflavone-Induced Adult Hen Liver" (Archives of Biochemistry and Biophysics, 282 (1) 170-182 (1990)) report on the purification and characterization of pure cytochrome P450. Characterization takes place by proteinase treatment using chymotrypsin under denaturing conditions.

Cohen, Ferré-D'Amaré, Burley and Chait in a paper entitled "Probing the solution structure of the DNA-binding protein Max by a combination of proteolysis and mass spectrometry" (Protein Science (1995), 4:1088-1099) propose a simple biochemical method that combines enzymatic proteolysis and matrix-assisted laser desorption ionization mass spectrometry to probe the solution structure of DNA-binding proteins. The method is based on inferring structural information from determinations of protection against enzymatic proteolysis, as governed by solvent accessibility and protein flexibility.

WO-A-2014082733 discloses a limited proteolysis (LiP) protocol, i.e. a method for the detection of the conformational state of a protein contained in a complex mixture of further proteins and/or other biomolecules, in particular in a complex native biological matrix, as well as assays for such a method. The method comprises, if needed after an extraction and/or lysis step, the following steps: 1. Limited proteolysis of the complex mixture under a condition where the protein is in the conformational state to be detected leading to a first fragment sample; 2. Denaturation of the first fragment sample to a denaturated first fragment sample; 3. Complete fragmentation of the denaturated first fragment sample in a digestion step to a completely fragmented sample; 4. Analytical analysis of the completely fragmented sample for the determination of fragments characteristic of having been the result both the limited proteolysis of step 1(corresponding to step D of the method according to the present invention) as well as of the complete fragmentation in the digestion step F for the determination of the conformational state.

Schopper et al in a paper entitled "Measuring protein structural changes on a proteome-wide scale using limited proteolysis-coupled mass spectrometry" (nature protocols, VOL.12 NO.11, 2017, 2391ff) report on protein structural changes induced by external perturbations or internal cues which profoundly influence protein activity and thus modulate cellular physiology. Limited proteolysis-coupled mass spectrometry (LiP-MS) is reported to be a recently developed proteomics approach that enables the identification of protein structural changes directly in their complex biological context on a proteome-wide scale. After perturbations of interest, proteome extracts are subjected to a double-protease digestion step with a nonspecific protease applied under native conditions, followed by complete digestion with the sequence-specific protease trypsin under denaturing conditions. This sequential treatment generates structure-specific peptides amenable to bottom-up MS analysis. Next, a proteomics workflow involving shotgun or targeted MS and label-free quantification is applied to measure structure-dependent proteolytic patterns directly in the proteome extract. Possible applications of LiP-MS include discovery of perturbation-induced protein structural alterations, identification of drug targets, detection of disease-associated protein structural states, and analysis of protein aggregates directly in biological samples. The approach also enables identification of the specific protein regions involved in the structural transition or affected by the binding event.

Jafari et al in a paper entitled " The cellular thermal shift assay for evaluating drug target interactions in cells" (nature protocols, VOL.9, NO.9, 2014, 2101ff) report thermal shift assays used to study thermal stabilization of proteins upon ligand binding. Such assays have been used on purified proteins in the drug discovery industry and in academia to detect interactions. A proof-of-principle study was published describing the implementation of thermal shift assays in a cellular format, which they call the cellular thermal shift assay (CETSA). The method allows studies of target engagement of drug candidates in a cellular context, exemplified with experimental data on the human kinases p38a and ERK1/2. The assay involves treatment of cells with a compound of interest, heating to denature and precipitate proteins, cell lysis, and the separation of cell debris and aggregates from the soluble protein fraction. Whereas unbound proteins denature and precipitate at elevated temperatures, ligand-bound proteins remain in solution. They describe two procedures for detecting the stabilized protein in the soluble fraction of the samples. One approach involves sample workup and detection using quantitative western blotting, whereas the second is performed directly in solution and relies on the induced proximity of two target-directed antibodies upon binding to soluble protein. The latter protocol has been optimized to allow an increased throughput, as potential applications require large numbers of samples.

Cappelletti et al in a paper entitled "Dynamic 3D proteomes reveal protein functional alterations at high resolution in situ" (Cell 184, 545-559, January 21, 2021) report that a global protein structural readout can be based on limited proteolysis-mass spectrometry (LiP-MS) which detects many functional alterations, simultaneously and in situ, in bacteria undergoing nutrient adaptation and in yeast responding to acute stress. The structural readout, visualized as structural barcodes, captured enzyme activity changes, phosphorylation, protein aggregation, and complex formation, with the resolution of individual regulated functional sites such as binding and active sites. Comparison with prior knowledge, including other 'omics data, showed that LiP-MS detects many known functional alterations within well-studied pathways. It suggested distinct metabolite-protein interactions and enabled identification of a fructose-1,6-bisphosphate-based regulatory mechanism of glucose uptake in E. coli. The structural readout dramatically increases classical proteomics coverage, generates mechanistic hypotheses, and paves the way for in situ structural systems biology.

Savitzki et al in a paper entitled " Tracking cancer drugs in living cells by thermal profiling of the proteome" (sciencemag.org, 3 October 2014, VOL 346 ISSUE 6205) report on performing thermal proteome profiling (TPP) on human K562 cells by heating intact cells or cell extracts and observed marked differences in melting properties between the two settings, with a trend toward increased protein stability in cell extract. Thermal profiling of cellular proteomes is reported to enable the differential assessment of protein ligand binding and other protein modifications, providing an unbiased measure of drug target occupancy for multiple targets and facilitating the identification of markers for drug efficacy and toxicity. Schuetz et al. in a paper entitled "Kinetics for Drug Discovery: an industry-driven effort to target drug residence time" (Drug Discovery Today, Vol. 22 (6), P. 896-911) report, that a considerable number of approved drugs show non-equilibrium binding characteristics, emphasizing the potential role of drug residence times for in vivo efficacy. Therefore, a detailed understanding of the kinetics of association and dissociation of a target-ligand complex might provide crucial insight into the molecular mechanism-of-action of a compound. This deeper understanding will help to improve decision making in drug discovery, thus leading to a better selection of interesting compounds to be profiled further. In the review, they highlight the contributions of the Kinetics for Drug Discovery (K4DD) Consortium, which targets major open questions related to binding kinetics in an industry-driven public-private partnership.

### SUMMARY OF THE INVENTION

### Drug Residence Time concept.

There is a fundamental difference between drug-target interactions in a closed (in vitro) system compared with in an open (in vivo) system. In in vitro systems, the Target, its substrates, and the drug are present at fixed concentrations, whereas in vivo, the concentrations of the drug, the Target, and its substrates can vary considerably spatiotemporally. Thus, measurements of equilibrium binding, such as Kd or Ki/IC50, reflect the concentration of the drug-target complex and thus potency in vitro, but they will not accurately predict in vivo pharmacodynamics. Because a considerable amount of all approved drugs exhibit non-equilibrium characteristics, it has been advocated that drug residence time could be more important for in vivo efficacy than in vitro equilibrium binding affinity. The accurate identification of drug-target interactions (DTIs) remains a decisive turning point in the discovery of new findings and in the understanding of the binding process. Thus, DTI identification is vital for the development of new drugs, optimizing the whole process chain and leveraging the information available for drug repositioning.

Retrospective studies have indicated that drugs with longer residence times have increased efficacy and fewer side effects because they occupy a higher fraction of their target over a longer period of time, even after clearance from systemic circulation. Residence time has become a key parameter for hit prioritization and lead optimization in many small-molecule drug discovery programs. Residence time can vary from less than a minute to several days, with covalently bound inhibitors representing the extreme, and can affect efficacy in different ways.

Despite the efforts to optimize drug-target affinity and specificity during drug development, more than 90% of small-molecule drugs fail in clinical settings. Hence, efforts have been taken to identify additional in vitro parameters that affect drug pharmacodynamic in vivo.

Besides unexpected toxicity, a lack of in vivo efficacy is often observed for many compounds. Such compounds appear promising in early drug discovery programs but fail in later clinical trials. One of the reasons for this is the increasing evidence that kinetic parameters seem to correlate much better with efficacy than affinity does.

In traditional in vitro methods, drug-target interactions have mostly been treated in terms of affinity measures or by means of static crystal structures of the bound complex [Schuetz, D.A., et al, Drug Discov Today, 2017. 22(6): p. 896-911]. The residence time concept, however, also takes into account the conformational dynamics of the protein which affect drug binding and unbinding. Thus, it considers the residence time of the drug-target complex rather than the binding affinity as the major contribution to in vivo pharmacological activity. In recent years, it has become evident that in vitro information on drug-target binding kinetics is of the utmost importance in candidate compound selection. However, in vitro information should be accompanied by information on the in vivo context in which the drug needs to exert its ultimate effect.

Drug-binding kinetics has gained interest among the drug discovery community due to reports stating that the efficacy of new drugs can be predicted by its binding and unbinding kinetics. Consequently, the in vitro dissociative half live was proposed as an important parameter for compound optimization.

Binding affinity provides information on the strength of the interaction between a drug-target (DT) pair and it is usually expressed in measures such as dissociation constant (Kd), inhibition constant (Ki) or the half maximal inhibitory concentration (IC50). IC50 depends on the concentration of the target and ligand (Cer et al., 2009) and low IC50 values signal strong binding. Similarly, low Ki values indicate high binding affinity. Kd and Ki values are usually represented in terms of pKd or pKi, the negative logarithm of the dissociation or inhibition constant.

The binding kinetics of a drug on its protein target is characterized by the bimolecular association rate constant (kon), which is the rate the drug binds, and the dissociation rate constant (koff), which is the rate of unbinding.

koff is an important parameter that regulates the time during which the drug is active. Whereas the calculation of the binding affinity is by now routine, the calculation of koff has proven more challenging because the timescales involved far exceed the limits of standard molecular dynamics simulation.

The ability to predict the mechanisms and the associated rate constants of protein-ligand unbinding is of great practical importance in drug design.

The drug-target residence time (τ) is the length of time that a drug remains bound to its target, and is defined mathematically as the reciprocal of the dissociation rate constant (1/koff), which can be estimated experimentally based on half-life of the drug-target complex.

Traditionally, the methods to measure drug-binding kinetics and thereof drug-residence time can be classified into three categories': labeled ligand methods, label-free methods and enzyme activity assays. Yet these methods have the major drawback of being based on "biased" analysis of purified anticipated drug-targets, as they use purified proteins or only specific groups of proteins, which are assumed to be the major targets of the drugs of interest. Consequently, these approaches disregard the evaluation of potential off-targets, since during protein purification the protein of interest is extracted from a complex protein sample, while the rest of the proteome is discarded. Thus, other proteins that may bind to the drug, and even have high residence time, will be absent of the reaction mixture, and therefore their potential interaction with the drug will not be considered.

Below, methods to measure drug-residence time are described.

### Labeled ligand methods.

The approach for labeled-ligand based methods is to incubate the target protein of interest with a test drug for a determined time period, and then remove the unbound drug using a filtration step. The drug-bound protein is then incubated with a radio-labelled version of the ligand, which bind to the protein once the unlabeled ligand is displaced. By measuring the association rate of the radioligand over time, it is possible to measure the dissociation rate of the unlabeled ligand, and therefore the residence time. These methods require production of a radioactive-labelled drug, which can become a costly process, and potentially not easily accessible. Furthermore, the disposal regulations for radio contaminated samples are undesirable for many laboratories. Hence, this type of methodologies are difficult to implement.

### Fluorescence-based methods.

An alternative to radioligand binding assays, is the use of fluorescence to measure binding kinetics. In this case, the ligand of interest is labelled with a fluorophore, which emits fluorescence at distinct wavelengths if the ligand is in solution, or if it binds to the target protein. Thus, by measuring the emitted fluorescence over time it is possible to understand the dynamics of binding-unbinding of the ligand and the protein of interest. This methodology avoids the need of radio-labelled ligands, however the addition of a fluorophore to the ligand can change its binding properties.

Other alternative is the use of fluorescent tracers that bind specifically to a set of proteins of interest (eg. Kinases). In either case, the labeled ligand methods require the development of different reagents and different ligands/engineered proteins for each assay, which can be costly and time consuming (eg. synthesis of a radiolabeled drug, conjugation with a fluorophore, development of fluorescent ligands). Furthermore, these methods use previously purified proteins, which are assumed to be the main targets of the ligand. This hinders the identification of potential off-targets, as the reactions occur with a small set of proteins in solution.

### Label-free methods.

Several label-free methods are possible for measuring drug-binding kinetics, thereby avoiding the hurdles associated with label-based methods.

### Surface plasmon resonance.

Surface plasmon resonance (SRP) is an analytical technique for studying molecular interactions, and it is the most widely-used label-free method for determination of small molecule binding kinetics. In SRP, the protein or proteins of interest are initially immobilized in the surface of a biosensor chip. Next, the ligand is injected over the chip under continuous flow, allowing the binding of the molecule to the protein target. Afterwards, buffer flows over the chip and the dissociation of the protein-ligand complex is monitored by detecting the change in refractive index in the surface of the chip, which is dependent on the increase in the mass of the surface upon binding of the ligand. SPR can be used, for example, to study the interaction between two proteins, a protein and an antibody, DNA and a protein, among other applications. Despite the flexibility of SPR to study interaction between different types of molecules, SPR is mostly suited for the characterization of the binding kinetics to purified immobilized proteins, which hinders the application of the method in an unbiased manner. Moreover, the conditions for protein immobilization have to be optimized to preserve the protein native state and not compromise its structure, conformation and binding-site accessibility, which can be a troublesome procedure.

### Acoustic biosensors.

Acoustic biosensors are based on quartz crystal resonators, in which the mode of oscillation depends on the cut and geometry of the quartz crystal. If mass is applied on to the surface of the quartz resonator, the frequency of the oscillation decreases. By measuring the change of frequency, it is possible to determine the change in mass, thereby allowing the detection of ligand-binding events to target molecules immobilized to the crystal. Similarly to previous methods, acoustic biosensors use purified proteins, thereby hindering an unbiased analysis of ligand targets.

### Bio-Layer Interferometry.

Bio-Layer Interferometry (BLI) is an optical technique for measuring macromolecular interactions by analyzing interference patterns of white light reflected from the surface of a biosensor tip, to which the molecule of interest is immobilized. By detecting changes in the patterns of the reflected white light, the method allows to monitor the kinetics and affinity of molecular interactions between the immobilized protein and a ligand. Consequently, this method also focus on the interaction of one pre-stablished protein of interest with another entity of interest, therefore not allowing it to be applied in an unbiased discovery manner, as the method lacks protein-level resolution if applied to a complex proteome.

### Resonant acoustic profiling.

Acoustic biosensors allow the label-free detection of molecules and the analysis of binding events. In general, they are based on quartz crystal resonators, in which the mode of oscillation depends on the cut and geometry of the quartz crystal. If mass is applied on to the surface of the quartz resonator, the frequency of the oscillation decreases. By measuring the change of frequency, it is possible to determine the change in mass, therefore it is possible to measure the binding of a ligand to a molecule immobilized to the quartz crystal. Yet, similarly to the methods described above, the resonant acoustic profiling method also requires the immobilization of target proteins of interest to the surface of the quartz crystal, thus hindering the unbiased identification d of protein-ligand interactions.

### Enzymatic activity assays.

When the protein of interest is an enzyme and there is a suitable assay available to monitor the enzymatic activity, the enzymatic reaction can be used to monitor the binding kinetics between the enzyme and the ligand, since the fraction of enzyme-bound complex is proportional to the enzyme activity.

These methods monitor the generation/consumption of a reaction product, generally by spectroscopic/fluorometric measurement, therefore avoiding the drawbacks of the label-based methods, and the need of immobilized proteins and specialized spectroscopic instrumentation for the label-free methods describe above.

### Jump-dilution method.

The jump-dilution method is the most widely-used enzyme activity assay to measure ligand-binding kinetics. In this method, the target enzyme is first incubated with a saturating concentration of the drug of interest, allowing the formation of the enzyme-ligand complex. Next, the reaction volume is increased to reduce the concentration of the ligand in solution, reducing the binding kinetics. The enzymatic activity is then monitored over time, and an equation is fitted to the progression curve to derive the residence time.

Despite the ease of implementation and the advantages of the jump-dilution method, it can only be applied to enzymes, thus highly reducing the range of protein targets. Furthermore, it also relies on the activity of a purified/previously anticipated enzyme/set of enzymes, thus hindering the identification of off-targets in an unbiased manner.

### Jump-dilution-Limited proteolysis methodology.

Since the jump dilution concept relies on the increment of reaction volume to reduce the concentration of the drug in the reaction mixture as a mean to reduce the binding kinetics, it maintains the native state of the proteome, a fundamental condition for the application of LiP-MS. Hence, by coupling the analytical potential of the LiP-MS/DarkLip-MS technologies with the jump dilution method, the inventors describe a method to probe drug-protein interactions at the proteome level. This way, the method here described does not rely on purified proteins, and thus analyzes at the same time all the potential binding partners for the test drug. Thereby, it overcomes simultaneously the limitations of the label-based and label-free methods described above (in particular, the need of immobilizing/labelling previously purified and anticipated drug targets) and the limitation of the jump-dilution method towards proteins with enzymatic activity. Hence, the present invention describes a method for unbiased and proteome-wide measurement of drug-residence times.

The Kinetics for Drug Discovery (K4DD) consortium in a paper entitled " Kinetics for Drug Discovery: an industry-driven effort to target drug residence time." (Drug Discov Today, 2017. p. 896-911) provided an updated report addressing drug-binding kinetics following a multidisciplinary approach.

Doing a review of actual existing drug-binding kinetics methods, among which surface plasmon resonance (SPR), Confocal microscopy, methods using an energy-transfer-based technique such as bioluminescence resonance energy transfer (BRET) or time-resolved fluorescence resonance energy transfer (TR-FRET), the authors concluded that 'for decades, drug discovery and development has focused on optimizing binding affinity while essentially neglecting drug-binding kinetics. In recent years, it has become evident that in vitro information on drug-target binding kinetics is of utmost importance in candidate compound selection. However, in vitro information should be accompanied by information on the in vivo context in which the drug needs to exert its ultimate effect.'

The method to determine the residence time between at least one Target and at least one Ligand according to the invention provides answers to this long felt need as it tends towards an in vivo context analysis using Targets contained in a complex mixture of further proteins and/or other biomolecules and/or a complex native biological matrix. Drug Target Residence Time is a key parameter for hit prioritization and lead optimization in many small-molecule drug discovery programs.

The inventors have surprisingly found that quantitative mass spectrometry-based techniques as LiP-MS^{™} or DarkLiP-MS^{™} that exploit structural proteomics for a variety of applications ranging from basic biology to Target deconvolution and biomarker discovery can be used to evaluate the Drug Target Residence Time, for candidate compound selection, hit prioritization and lead optimization in many small-molecule drug discovery programs.

The method according to the invention allows:
a) to prioritize Target candidates based on their residence time.
   This is achieved in the LiP-MS and DarkLiP-MS approaches through preferably providing a list of potential drug-target candidates, ranked by a score derived from a computational analysis. The drug-residence time therefore allows to understand which of the drug-targets are the ones that are expected to have the longest interaction.
b) to select the best drug against a particular-protein/set of proteins.

Recent research suggests that drugs with higher residence time for the Target of interest are more efficient and translate into better results in vivo and during clinical trials. Drugs which present a high residence-time for the anticipated Target of interest, and with minimal residence time in other proteins (off-targets), will be considered as more specific, and less likely to lead to strong side effects. A correlation can be made between the drug Target residence time and the selectivity and or/ efficacy of the drug.

Hence, by measuring the drug-residence time for several drugs that aim to target the same protein/sets of proteins, the inventors are able to predict which one of the drugs is more probable to succeed in vivo.

c) to predict the in-vivo off Target / side effect of different drugs based on their binding kinetics.

Due to the complexity of biological systems, most drugs bind to more than one protein Target, in many cases affecting unpredicted off-Target proteins. The effect of a drug on its Targets depends on the drug-protein interaction, therefore longer interactions will promote longer effects. Measuring relative drug-residence times for the entire proteome allows to understand what proteins are the ones that interact for longer times with the drug, and thus predict what are the main targets/off-targets of the drug. The higher the number of protein Targets identified with high residence time, the higher the likelihood of strong side effects. Drugs with high residence times for the main Target, associated with low number of off-targets with long-lasting interactions, are more likely to be more specific and less likely to lead to strong side-effects. In this way the method according to the invention can be used to determine medically relevant Targets.

A major use of the method according to the invention relates to in-vitro comparison of drugs specificity. The present invention answers the long-felt need consisting in understanding and comparing the mechanism of action of several drugs, for instance providing informative readouts on the mechanism of action of early-phase drugs.

This is achieved by estimation of the drug-residence time among the Targets and drugs of interest.

The methods according to the invention allow to identify the drugs that are most likely to succeed in in-vivo trials, due to less off-targets relatively to the main Target.

In a first embodiment the invention provides a method to determine the residence time between at least one Target and at least one Ligand, said Target contained in a complex cellular mixture of further targets and/or other biomolecules comprising the following steps:
- Step A: Incubating said at least one Target with the at least one Ligand, for an appropriate incubation period of time,
- Step B: Stopping the binding reaction by removing the at least one Ligand (in as far as not bound to Target); or reducing the number of binding events (per unit time) by reducing the concentration of the Ligand (in as far as unbound to the Target),
- Step C: Incubating the at least one Ligand-bound to the at least one Target for different periods of time,
- Step D: Limited proteolysis of the complex mixture under a condition in which the at least one Target is in the original conformational state to be detected leading to a first fragment sample,

Then either the following steps are carried after Step D (if a Lip.MS protocol is used):
- Step E: Denaturation of the sample to a denaturated first fragment sample.
- Step F. Digestion of the sample to obtain a completely fragmented sample or the following step is carried out directly after step D (if a DarkLiP-MS protocol is used) Step G. Removal of large peptides and proteins or other biomolecules from said first fragment sample to form an enriched fragment sample;

This for both cases (for both LiP-MS and DarkLiP-MS protocols) is followed by:
- an analytical analysis, wherein quantitative mass spectrometry-based assays in the form of selected/parallel reaction monitoring (SRM/PRM) and/or data-independent acquisition of product ion spectra is used,
- for the case of Steps E and F (LiP-MS protocol), the analytical analysis is run on the completely fragmented sample for the determination of fragments characteristic being the result of both the limited proteolysis step D. as well as of the complete fragmentation Step.F
- for the case of Step G (DarkLiP-MS protocol) the analytical analysis is run on the enriched fragment sample,
- measurement of peptide abundance over distinct incubation times post Ligand removal, to determine the dissociation half-life.

The residence time (τ) is calculated according to equation 1, $τ = \frac{1}{koff} = \frac{t 1 / 2}{ln 2} = \frac{t 1 / 2}{0.693}$ wherein
*τ* = Residence time
k_{off} = dissociation constant
t_{1/2} = Dissociation half-life
and wherein for the case of Steps E and F (for LiP-MS protocol) the determination of the residence time between the Target and the Ligand is based on a quantitative comparison of the analytical analysis of
   - the completely fragmented sample with the analytical analysis of the completely fragmented control sample
wherein for the case of Step G (DarkLiP-MS protocol) the determination of the residence time between the Target and the Ligand is based on a quantitative comparison of the analytical analysis of:
   - the enriched fragment sample with the analytical analysis of the enriched fragmented control sample.

The complex cellular mixture of further proteins and/or other biomolecules can e.g. be a complex native biological matrix, or generally a biological probe such as body fluids (plasma, cerebrospinal, urine, etc.), can be based on tissue, an environmental sample (e.g. sea water, etc.), biological secretions etc.

In another embodiment of the method according to the invention, the Limited proteolysis (step D) of the complex mixture is carried out over a time span of 1-60 minutes, preferably in the range of 2-30 minutes, or 2 - 10 minutes or 2 - 5 minutes, further preferably at a temperature in the range of 20-40°C.

In another embodiment of the method according to the invention, in LiP-MS protocol in the digestion step Trypsin is used, preferably at a temperature of 15 -70°C, over a time span of 2 to 24 hours, at an enzyme to substrate weight ratio in the range of 1/10.

In another embodiment of the method according to the invention, the Target is a protein or at least a portion of a proteome under native condition and wherein the Ligand comprises one of the following entities: a small drug compound, a natural occurring protein cofactor, coenzyme or co-substrate (e.g. metal ion, organic molecule), an amino acid, a synthetic peptide-drug, a natural occurring peptide, a macromolecule (i.e. another protein, nucleic acid, lipid or macromolecular complex).

In another embodiment of the method according to the invention, the at least one Target is a protein based exclusively on proteinogenic amino acids or is based on proteinogenic amino acids and carries post-translational modifications and the complex mixture of further proteins and/or other biomolecules is a complex native biological matrix.

In another embodiment of the method according to the invention, in the limited proteolysis step the proteolytic system is selected from the group consisting of proteinase K, thermolysin, subtilisin, pepsin, papain, α-Chymotrypsin, elastase, and mixtures thereof.

In a further embodiment of the method according to the invention, in the limited proteolysis step the proteolytic system is used at a concentration, with respect to the total biomolecular content in the sample, given as the ratio of enzyme to biomolecular content, in the range of 1/25-1/10000, preferably in the range of 1/50-1/1000 by weight.

In a yet further embodiment of the method according to the invention, when DarkLiP-MS protocol is used the removal ( step G) of large peptides and proteins is done in a filtration, separation or enrichment step, including size filtering; chromatography including size exclusion, hydrophobic or anion exchange chromatography; physical removal including phase separation, absorption, precipitation; filtration, separation or enrichment based on hydrophilic/hydrophobic properties; filtration, separation or enrichment based on electric/magnetic field; or a combination thereof.

In another embodiment of the method according to the invention, when DarkLiP-MS protocol is used peptides, proteins and/or other biomolecules having a molar weight larger than 20 kDa, preferably having a molar weight larger than 15 kDa, most preferably having a molar weight larger than 10 kDa are removed (step G) from the first fragment sample.

In another embodiment of the method according to the invention, before analytical analysis step, a proteomics workflow, in particular involving denaturation, C18 cleanup, or a combination thereof is carried.

In another embodiment of the method according to the invention, for the analytical analysis step SWATH-MS and/or optionally data-dependent acquisition (shotgun), is used.

In another embodiment of the method according to the invention, for quantitative mass spectrometry-based assays heavy labelled fragments characteristic of being the result of the limited proteolysis or of the complete fragmentation are spiked into the original complex mixture or into the completely fragmented sample.

In a further embodiment of the method according to the invention, for quantitative mass spectrometry-based assays heavy labelled fragments characteristic of being the result of the limited proteolysis of step D. as well as remaining after the removal of large peptides and proteins or other biomolecules of step G, are spiked into the original complex mixture and/or into the first fragment sample and/or into the enriched fragment sample.

In another embodiment, the method according to the invention is used for at least one of the following:
a) to prioritize Target candidates based on their residence time, or
b) to select the best drug against a particular-protein/set of proteins, or
c) to predict the in-vivo off Target / side effect of different drugs based on their binding kinetics, or
d) to determine medically relevant Targets.

In another embodiment the invention provides a method aiding in the prognostic or diagnosis of disorders pathologies in patients early in the progression of the dysfunction /disease comprising:
a) determining the presence of at least one selected Target protein and
b) determining the residence time between at least said selected Target protein and at least one Ligand in a patient's biologic sample such as plasma, serum, cerebrospinal fluid or urine and
c) comparing the concentration data to concentration data from populations affected by this disorder/ disease to verify or nullify the presence of the given pathologies.

Possible experimental set up with the DarkLiP-MS approach: proteins-drug incubation and proteolysis.
- Protein extracts/purified proteins are incubated with the drug of interest for a defined period of time (for ex. 10min) in triplicates.
- The jump dilution method is used to reduce binding of the drug to its Targets, by increasing the solution volume (for example, 100 times).
- Each sample is incubated by a defined time post-dilution (for ex. 0 minute to 1hour)
- A limited proteolysis step is performed on the native cellular extracts, using the enzyme thermolysin and the DarkLiP-MS methodology.
- Thermolysin is inhibited by EDTA, and the generated partially fragmented sample is purified with a C18 resin, which removes large peptide and intact proteins. The generated fragmented sample is analyzed by mass spectrometry, and compared to a sample without the drug (treated with DMSO).

Possible experimental setup with the LiP-MS approach: proteins-drug incubation and proteolysis.
- Protein extracts/purified proteins are incubated with the drug of interest for a defined period of time (for ex. 10min) in triplicates.
- The jump dilution method is used to reduce binding of the drug to its Targets, by increasing the solution volume (for example, 100 times).
- Each sample is incubated by a defined time post-dilution (for ex. 0 minute to 1hour)
- A limited proteolysis step is performed on the native cellular extracts, using the enzyme thermolysin and the LiP-MS methodology.
- Thermolysin is inhibited by EDTA, and the generated partially fragmented sample is dried to reduce the volume.
- The dried partially fragment sample is resolubilized and digested with LysC/Trypsin, generating a fully fragmented sample.
- The fully fragmented peptides are purified with a C18 resin, analyzed by mass spectrometry, and compared to a sample without the drug (treated with DMSO). Implementation LiP-MS/ DarkLiP-MS with low protein concentrations.

In order to induce minimal effect on the maintenance of the proteome native state, the jump dilution method discussed above is coupled with LiP-MS to assess drug-residence times.

To test the effect of low protein concentrations on the limited proteolysis, cellular extracts are incubated with the drug (e.g. staurosporine) at the standard concentration of the cellular extract, and the volume will be increased with the addition of the enzyme (the concentration of the drug will be kept constant).

### LiP-MS / DarklLiP-MS technologies to assess drug-target residence time.

LiP MS and DarkLiP-MS techniques enable MS-based identification of unique structural and/or /conformational states of proteins and/or structural and/or /conformational protein changes in complex biological or clinical specimens with high sensitivity, coverage and throughput using LC-MS, DIA (data-independent acquisition of product ion spectra) mass spectrometry and unspecific database searches.

Identifying such unique protein conformational changes and states provides valuable information about protein structure and function, enables the identification of targets of drugs or metabolites of interest, contributes to the characterization of biochemical and signaling pathways involved in the response to the perturbation and informs on disease mechanisms.

To illustrate that LiP-MS/ DarkLiP-MS methodologies allow to monitor the decay of drug-induced structural changes upon drug dilution, cell extracts are incubated with drugs for which the residence time has been previously measured, and LiP-MS is performed at distinct time points after a 100-fold jump dilution.

A potential optimal control drug is the pan-CDK inhibitor Roniclib which shows kinetic specificity for CDK2 and CDK9 (despite IC50 values in the nanomolar range towards CDK1, CDK2, CDK4, CDK6, CDK7 and CDK9). Staurosporine residence time is also evaluated, as half-life in the minute range towards certain kinases has already been described.

Finally, irreversible inhibitors as AEBSF and E64 (general inhibitors of serine and cysteine proteases, respectively) should lead to "infinite" residence times.

### Relative assessment of drug-residence times between several compounds.

To illustrate that LiP-MS/ DarkLiP-MS can be used to predict the in-vivo efficacy of different drugs based on their binding kinetics, FDA-approved compounds are compared with compounds known to induce strong side effects. Thereby, it is possible to determine whether the clinical or pre-clinical failure of the drug can be predicted by the drug-residence time in off-target proteins.

### LIP-MS technology.

Limited proteolysis mass spectrometry (LiP-MS) technology used in the present invention (designated as LiP protocol) is described in WO-A-201408273, the content of which is incorporated here by reference.

WO-A-2014082733 discloses a limited proteolysis (LiP) protocol, i.e. a method for the detection of the conformational state of a protein contained in a complex mixture of further proteins and/or other biomolecules, in particular in a complex native biological matrix, as well as assays for such a method. The method preferably comprises, if needed after an extraction and/or lysis step, the following steps:
- step 1 (corresponding to step D of the method according to the present invention): limited proteolysis of the complex mixture under a condition where the protein is in the conformational state to be detected leading to a first fragment sample;
- step 2 (corresponding to step E of the method according to the present invention): denaturation of the first fragment sample to a denaturated first fragment sample;
- step 3 (corresponding to step F of the method according to the present invention): complete fragmentation of the denaturated first fragment sample in a digestion step to a completely fragmented sample;
- step 4: analytical analysis of the completely fragmented sample for the determination of fragments characteristic of having been the result both the limited proteolysis of step 1. as well as of the complete fragmentation in the digestion step 3. for the determination of the conformational state.

### Limited proteolysis (LiP) and advanced targeted mass spectrometry workflow.

The proposed method is preferably based on the coupling of a biochemical technique called limited proteolysis (LiP) and an advanced targeted mass spectrometry workflow, involving selected reaction monitoring (SRM) or SRM-like approaches (such as SWATH-MS) or other mass spectrometry approaches such as Parallel Reaction Monitoring (PRM), Data-Dependent Acquisition (DDA), isobaric labelling quantification or untargeted analysis of DIA. Liquid chromatography coupled to Mass Spectrometry (LC-MS) has been used for many years in the proteomic community for the identification and quantification of peptides (and thus proteins) from complex sample mixtures. The commonly most used approaches are variants of the so-called LC-MS/MS or "shotgun" MS approach that is based on the generation of fragment ions from precursor ions that are automatically selected based on the precursor ion profiles (data dependent analysis, DDA). The most mature technology is called selected Reaction Monitoring (SRM), frequently also referred to as multiple reaction monitoring (MRM). The targets for MRM experiments are defined on a rational basis and depend on the hypothesis to be tested in the experiment. Selected combinations of precursor ions and fragment ions (so called transitions, the set of transitions for one target precursor is called MRM assays) for these targets are programmed into a mass spectrometer, which then generates measurement data only for the defined targets. Another variant of targeted proteomics is data independent acquisition, and a more recently presented variant commonly called SWATH-MS approach. Here, the targeted aspect is introduced only on the data analysis level. Contrary to MRM, this approach does not require any preliminary method design prior to the sample injection. Since the LC-MS acquisition covers the complete analyte contents of a sample through the entire mass and retention time (RT) ranges the data can be mined a posteriori for any peptide/precursor of interest. Data is acquired in a data independent manner, on the complete mass range (e.g. 200-2000 Thomson) and through the entire chromatography, disregarding of the content of the sample. This is commonly achieved by stepping the selection window of the mass analyzer step by step through the complete mass range. In effect, this data acquisition method generates a complete fragment ion map for all the analytes present in the sample and relates the fragment ion spectra back to the precursor ion selection window in which the fragment ion spectra were acquired. This is achieved by widening the precursor isolation windows on the mass analyzer and thus accounting a priori for multiple precursors co-eluting and concomitantly participating to the fragmentation pattern recorded during the analysis. Such a precursor window is called a swath. The result is complex fragment ion spectra from multiple precursor fragmentations, that require a more challenging data analysis. Unlike in shotgun proteomics, for the MRM and SWATH technology spectra are repeatedly recorded for the same analytes with a high time resolution. The high time resolution when compared to shotgun proteomics, together with the limited fragment ion information for MRM and the limited fragment ion to precursor ion association for SWATH, makes a completely new type of data analysis necessary. Since only a limited number of pre-defined analytes are being monitored, it is not necessary to make a shotgun proteomics type database search by comparing the spectra to a complete theoretical proteome. Instead, a number of scores have been described that are based on signal features such as shape, co-elution of transitions, and similarity of transition intensities to assay libraries. Furthermore, confidence estimation of identification in MRM by means of false discovery rates cannot be done as for the classical shotgun proteomics. Therefore, a novel approach has been developed by measuring transitions for non-existing peptides (decoy transitions) (Reiter L, Rinner O, Picotti P, Huttenhain R, Beck M, Brusniak MY, Hengartner MO, Aebersold R: mProphet: automated data processing and statistical validation for large-scale SRM experiments. Nature methods 2011, 8(5):430-435). The data from these decoy transitions can be used to derive false discovery rates as is done in shotgun proteomics. This confidence estimation by means of false discovery rate is necessary to determine the data significance level and allow user defined quality filtering of the data. SWATH data are distinct from MRM data. In contrast to MRM, full fragment ion spectra are recorded using the SWATH method. The time resolution is usually chosen similarly as in MRM. When comparing SWATH with shotgun proteomics, the difference is that in SWATH the fragment ion spectra are derived from a much higher number of precursors because the window for precursor selection is usually chosen as high as 25Th instead of roughly 1Th for shotgun proteomics. This high complexity of the fragment ion spectra makes it unpractical to analyze the data as in shotgun proteomics using database searches. However, the data can be analyzed similarly to MRM data with the additional benefit of the high time resolution in the data. This can be done by extracting ion currents corresponding to transitions in MRM. The resulting data can then be analyzed very similarly to MRM. In all variants of LC coupled mass spectrometry, proteins in samples for MRM experiments are digested into smaller peptides prior to the analysis. The resulting peptide mixture is usually chromatographically separated in order to reduce the complexity of the sample. Chromatographic separation adds a time dimension to the recorded data of the mass spectrometer, the retention time (RT). Data independent acquisition data can also be analyzed in a spectrum-centric or untargeted fashion where queries are made in the search space based on the data, for instance based on the precursor ion (MS1) signals in the data (unlike for SWATH). This is the same analysis type that is typically used for DDA. Further, various quantification technologies can be used such as isobaric labelling such as TMT or iTRAQ or stable isotope labeling with amino acids in cell culture (SILAC) or isotopically heavy labelled peptides can be added for the absolute quantification of peptides and proteins. Also parallel reaction monitoring (PRM) can be used which is similar to MRM but it is performed on a high-resolution instrument and fragment ion scans (MS2 scans) are acquired in full range for the analyte(s) that are targeted in the analysis.

SRM assays are specific, quantitative mass spectrometry-based assays for proteins of interest, akin to antibodies for Western blotting, but with higher multiplexing capabilities and lower development time (assays for 100 peptides can be developed in one hour). We previously demonstrated that SRM allows quantifying proteins in a broad range of cellular abundances, down to <50 copies per cell, in total cell lysates (see P Picotti et al., Cell 138 (4), 795 (2009); and Picotti at al. Nature Methods, VOL.9 NO.6, JUNE 2012, these references are, as concerns the SRM technique specifically included in the disclosure), resolving proteins with high (>95%) sequence overlap and measuring target peptides across large numbers of samples. Therefore, this technology enables quantitative measurements of specific peptides in very complex samples. Recently, further developments of the SRM approach include SRM-like approaches based on data-independent acquisition of product ion spectra and their targeted analysis (SWATH method, see LC Gillet et al., Mol Cell Proteomics 11 (6), 0111 016717 (2012), the disclosure of which is included as concerns the SWATH method and the data extraction).

### DarkLiP-MS technology.

DarkLiP-MS technology used in the present invention (also termed DarkLiP protocol) is described in EP21212313.7 'method and tools for the determination of conformations and conformational changes of proteins and of derivatives thereof ', Biognoys AG filed on 03 December 2021, the content of which is incorporated here by reference.

DarkLiP-MS approach is a novel complementary approach to LiP-MS that can provide entirely unique information from a traditional LiP-MS experiment because it focuses on generating and analyzing a unique set of peptides. By digesting only native proteins for a limited time, followed by the introduction of a filtration/enrichment step, the proposed technique reduces the number of information-poor peptides and/or protein pieces that are problematic during sample preparation, data acquisition and analysis. This boost in the signal-to-noise ratio can represent a significant improvement in data quality and subsequently in biological insights.

More generally speaking, DarkLiP-MS approach relates to a method for the detection of the conformational state of at least one protein, said at least one protein being contained in a complex mixture of further proteins and/or other biomolecules (such a complex mixture can e.g. be a complex cellular extract mixture, or generally a biological probe such as body fluids (plasma, cerebrospinal, urine, etc). Said at least one protein in said complex (cell extract) mixture has been subjected to a condition inducing a structural change in said at least one protein.

DarkLiP-MS approach also relates to a method for the detection of the conformational state of at least one protein, said at least one protein being contained in a tissue, an environmental sample (e.g. sea water etc.), biological secretions, e.g. obtained by cells lysed according LiP-MS protocol. Said at least one protein has been subjected to a condition inducing a structural change in said at least one protein.

The protein concentration can be determined using an assay kit.

The method preferably comprises, if needed after an extraction and/or lysis step, the following sequence of steps:
Step 1. (corresponding to step D of the method according to the present invention):
   limited proteolysis of the complex mixture (for example a cell extract mixture) under a condition in which the at least one protein is in the original conformational state to be detected leading to a first fragment sample; directly followed by
Step 2. (corresponding to step G of the method according to the present invention):
   removal of large peptides and proteins or other biomolecules from said first fragment sample to form an enriched fragment sample;

- Analytical analysis of the enriched fragment sample for the determination of fragments characteristic of having been the result of the limited proteolysis of step 1. as well as remaining after the removal step 2. for the determination of the conformational state of said at least one protein.

When mentioning "directly followed" at the end of step D., this excludes further denaturation and/or proteolysis steps but does not exclude further steps involved in the termination of the limited proteolysis of step D., i.e. steps of quenching the limited proteolysis by adding corresponding reagents (e.g. Deoxycholic acid sodium solutions), increasing temperature, washing, filtering, sedimentation, solvent and/or pH adjustments, or a combination thereof and the like. In other words, during the limited proteolysis digestion, there are no other steps that contribute to the peptide generation (e.g. denaturation to increase cleavage site access or addition of other proteases, etc) so step 1. from a digestion perspective is 'complete'. However, prior to step 2 there can be an addition of deoxycholate and bringing the temperature up to e.g. 98°C, which is done to stop the protease activity from step D.

DarkLiP-MS technology is a new variant of the LiP-MS approach that instead focuses on an increased relative ability to identify peptides that convey structural/conformational information from proteins at the expense of peptide (and thus protein) identifications that do not necessarily report structural information. By focusing the attention on enrichment of structurally informative peptides, the proposed technique increases the signal-to-noise ratio, thus making the identification of protein structural changes more robust.

The key feature of the proposed technique is that it increases the number and abundance of truly informative peptides relative to the total number of peptides that are contained in a sample, thus strongly reducing the dynamic range challenge inherent to proteomics samples. This problem is very pronounced for human body fluids such as blood plasma but also exists in samples where the protein(s) of interest are of low abundance such as is often the case for the study of drugs with single (or few) protein targets. Beside other factors, this broad dynamic range originates from the protein size distribution in combination with the distribution of peptide responses in the mass spectrometer. In a classical proteomic approach, including the LiP-MS approach, the larger a protein is, the more tryptic peptides it will generate. Hence there is a strong correlation between protein size and/or abundance, and the likelihood that such a protein will generate at least some peptides that have a strong response in the mass spectrometer. In contrast, the proposed technique approach produces peptides mainly from protease accessible regions of proteins that are in their native or near native conformation (i.e. not denatured). These peptides tend to be on the solvent-exposed surfaces of proteins. This substantially reduces the proportional number of peptides derived from large proteins since the relationship between protein surface area and volume is not a fixed ratio and on average decreases as a protein increases in size. By exploiting this surface area to size ratio bias the proposed technique aims at reducing the dynamic range inherent to proteomics samples that is due, at least in part, to the natural size distribution of proteins. In addition, the proposed technique focuses on the information rich accessible peptides that report on protein structure and protein structural changes.

The proposed technique exploits two key processes, namely a limited digestion coupled with enrichment of short, MS-compatible peptides. The collective intention of the procedure is to increase the number of peptides that convey conformational/structural information (signal), while decreasing the amount of peptides that do not contain such information (noise) in a mass spectrometer ready sample.

This is accomplished in a surprisingly simple and efficient approach by using a limited digestion step under non-denaturing (i.e. protein structure retaining) conditions, using a specific or non-specific protease. Limited digestion can be varied by modifying the enzyme to substrate ratio, performing the digest at lower temperature or by performing the digest on a relatively short time scale. The resulting peptide mixture is then, in contrast to the LiP-MS protocol, not fully denaturated and completely fragmented, but it is filtered or treated in other ways (see below) to remove large peptides/protein fragments, that represent a large fraction of the mixture and contain little information on the structure/conformation and/or structure/conformational changes of proteins. By removing large peptides/protein pieces from the digest prior to mass spectrometry, the proposed technique also reduces the number of non-informative peptides that could introduce artifacts and/or noise to the experiment from the perspective of peptide identification in the mass spectrometer and also during downstream data analysis. This removal can be achieved by a process that separates large peptides/protein pieces, to enrich for suitable peptides including size filtering (e.g. using a 10k MWCO filtration device), chromatography (e.g. size-exclusion, hydrophobic or anion exchange) or physical processes (e.g. phase separation, absorption or precipitation). Compared to classical LiP-MS, the proposed technique omits a full trypsinization step under denaturing conditions after the limited proteolysis step.

physical removal including phase separation, absorption, precipitation; filtration, separation or enrichment based on hydrophilic/hydrophobic properties; filtration, separation or enrichment based on electric/magnetic field; or a combination thereof. Filtration can also be performed with two or more filters such that a specific peptide size range is enriched, i.e. not only removing large peptides but also very small ones that will not be specific enough because of a very short amino acid sequence or that are not suited for mass spectrometric analysis.

In addition to single protease being used for the limited digestion step, the proposed technique can be augmented by the use of protease mixtures and/or sensitizers (e.g. heat, urea). Instead of protease mixtures two distinct proteases (or sets of proteases) can also be used on an aliquot of the sample and the sample pooled afterwards. Pooling can be done after digestion or separation of peptides from the rest of the sample. Instead of pooling, the samples can also be processed and measured separately completely. Protease mixtures and sensitizers act in two ways to improve identification of proteins of interest according to the proposed technique. Protease mixtures include proteases that target different amino acids for cleavage and thus quite simply enable both more and unique peptides to be generated during the digestion step. Sensitizers work by slightly disrupting the native state of the protein to enable novel protease cleavages. Sensitizers are particularly useful for the proposed technique when specific states are being investigated for changes in protein structure (e.g. with and without a drug or metabolite). In such cases the effect of sensitizers is magnified as proteins of interest will become more or less susceptible to the particular sensitizer if their structure has been changed (i.e. stabilized or destabilized) by an event such as binding of a drug.

In these ways, the proposed technique is able to identify specific protein conformations or structures by exploiting traditionally ignored peptides (i.e. peptides with two non-tryptic termini located at the surface of proteins). The step that removes large peptides and proteins (e.g. filtration) included in the proposed technique workflow although altering overall peptide/protein identification numbers, leads to a relative enrichment for structurally informative peptides.

The proposed technique workflow comprises or consists of the following steps:
Step A: A sample containing a protein or a proteome from cells, tissues, or body fluids is investigated for protein structure and/or conformational states. This includes but is not limited to incubation with a Ligand (e.g. small molecule, metabolite, etc.) at defined concentrations (treatment mixture), including control (vehicle) samples or subjecting the lysate to conditions that induce a structural state change such as temperature, metabolic stimulant, etc. including unstimulated samples. Whatever conditions are utilized, native protein structures (primary, secondary, tertiary and preferably / potentially also quaternary structure) are preserved throughout.
Step B: Stopping the binding reaction by removing the at least one Ligand in as far as unbound to the Target; or reducing the concentration of the Ligand in as far as unbound to the Target to reduce the number of binding events (per unit time).
Step C: Incubating the at least one Ligand bound to the at least one Target for different periods of time,
Step D: Each sample is subjected to a limited digestion step using a specific or an unspecific protease (or a combination thereof). This digest should be relatively short, typically 1-5 minutes, and rapidly quenched.
Step G: Following this digestion step, the larger peptides and protein fragments are removed e.g. via filtration, separation or enrichment devices (see methods above and below).

The remaining peptides are then processed using a standard proteomics workflow (e.g. denaturation, C18 clean-up, etc) and analyzed via LC-MS/MS so that the peptides can be identified and quantified.

Further embodiments of the invention are laid down in the dependent claims and also in the specification further below.

### BRIEF DESCRIPTION OF THE DRAWINGS

Preferred embodiments of the invention are described in the following with reference to the drawings, which are for the purpose of illustrating the present preferred embodiments of the invention and not for the purpose of limiting the same. In the drawings,
- Fig. 1: shows a schematic of the proposed approach; native proteins are incubated with a ligand at defined concentrations, including control (vehicle) samples; each sample is subjected to a brief rapidly quenched limited digest step; next, larger peptides and protein pieces are removed), yielding unique peptide populations dependent upon protein conformation during the limited digest; a standard proteomics workflow can be implemented using the remaining peptides;
- Fig. 2: shows the concept for measurement of drug-residence times by LiP-MS/DarkLiP-MS;
- Fig. 3: shows the experimental workflow used to measure drug-residence times by coupling the jump dilution method with LiP-MS;
- Fig. 4: shows the workflow for sample processing for analysis by mass spectrometry;
- Fig. 5: shows the expected results for measurement of drug-residence times by LiP-MS;
- Fig. 6: shows a proof-of-concept: The ligand Staurosporine and the target Dual specificity mitogen-activated protein kinase kinase 3 (MP2K3);
- Fig. 7: shows a proof-of-concept: The ligand Calyculin A and the target phoshatase PP2A-alpha (PPP2CA);
- Fig. 8: shows a proof-of-concept: The ligand Calyculin A and the non-target protein Kynureninase (KYNU).

### DESCRIPTION OF PREFERRED EMBODIMENTS

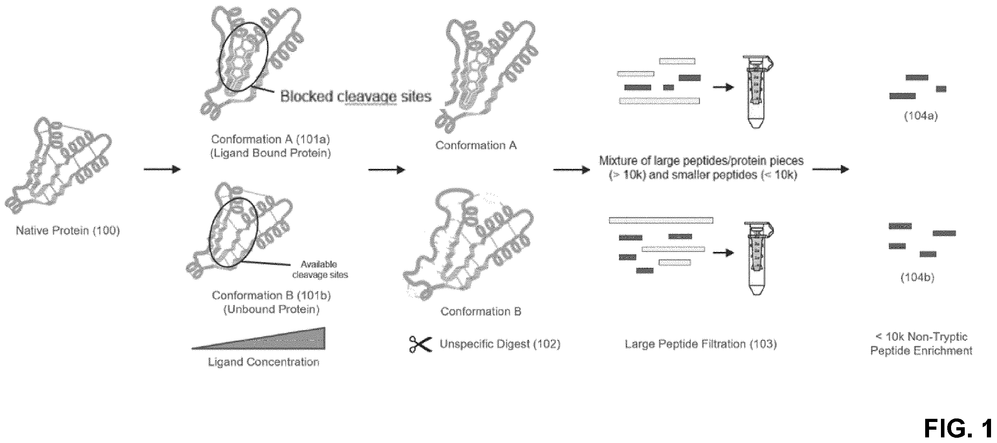

A schematic with an example of how the technique supports the detection of structural/conformational changes is shown in **Fig. 1****,** using the investigation of a ligand binding event as an example.

Fig. 1. shows a schematic of the DarkLiP-MS approach where the differentiating condition for the conformational difference between the reference and the altered sample is the addition of a ligand. Native proteins (100) are incubated in the altered sample with a ligand (101a) at defined concentrations (upper path), while no ligand is added in the control (vehicle) samples (101b) (lower path). Each sample is subjected to a brief (1-5 minutes), rapidly quenched limited digest step (102), typically with an unspecific protease. Next, larger peptides and protein pieces are removed (e.g. via filtration) in step (103), yielding unique peptide populations (104a/b) that are dependent upon protein conformation during the limited digest (102). A standard proteomics workflow can be implemented thereafter using the remaining peptides (e.g. denaturation, C18 clean-up, LC-MS and analysis).

The DarkLiP-MS approach is based on the coupling of a biochemical technique called limited proteolysis (LiP) and an advanced targeted mass spectrometry workflow, involving selected reaction monitoring (SRM) or SRM-like approaches (such as SWATH-MS) or other mass spectrometry approaches such as Parallel Reaction Monitoring (PRM), Data-Dependent Acquisition (DDA), isobaric labelling quantification or untargeted analysis of DIA. The analytical analysis technique used in the present invention based on quantitative mass spectrometry enables MS-based identification of unique structural/conformational states of proteins and/or structural/conformational protein changes in complex biological or clinical specimens with high sensitivity, coverage and throughput using LC-MS, DIA (data-independent acquisition of product ion spectra) mass spectrometry and unspecific database searches. The structural/conformational states of proteins sampled by the proposed approach can be natural, non-natural or a mixture of both, depending on the application. Implementations of this technique can be used to investigate protein structure under standard conditions, during protein binding to a drug/small molecule or metabolite, upon binding to other proteins (protein-protein interactions, i.e. protein complexes), upon binding to a variety of other molecules (e.g. lipids or DNA) as a result of a chemical modifications (e.g. PTMs, like protein phosphorylation), or a change of local environment (e.g. temperature increase, ionic strength changes or presence of chaotropes). The proposed technique allows the detection of proteins that undergo structural changes in a hypothesis-free manner when a perturbation is induced in the investigated system (e.g. triggers of immune signaling or disease). Identifying such unique protein conformational changes and states provides valuable information about protein structure and function, enables the identification of targets of drugs or metabolites of interest, characterizes biochemical and signaling pathways involved in the response to the perturbation and informs on disease mechanisms. Furthermore, altered protein structures can be used as a proxy for disease detection (i.e. structural biomarkers). The insights into the structural state and dynamics of the structural proteome provide a deeper understanding of both physiological and non-physiological mechanisms of action, both of which can represent major hurdles in advancing our understanding of diseases, as well as supporting drug design and refinement. Thus, the proposed technique enables exploiting structural proteomics for a variety of applications ranging from basic biology, to target deconvolution and biomarker discovery.

This way, the proposed approach is a novel complementary approach to LiP-MS that can provide entirely unique information from a traditional LiP-MS experiment because it focuses on generating and analyzing a unique set of peptides. By digesting only native proteins for a limited time, followed by the introduction of a filtration/enrichment step, the proposed technique reduces the number of information-poor peptides and/or protein pieces that are problematic during sample preparation, data acquisition and analysis. This boost in the signal-to-noise ratio can represent a significant improvement in data quality and subsequently in biological insights.

Limited proteolysis/ Removal -filtration step/ analytical analysis preferred embodiment.

According to a preferred protocol, in the limited proteolysis step D. a proteolytic system selected from the group consisting of proteinase K, thermolysin, Subtilisin, Pepsin, Papain, α-Chymotrypsin, Elastase, and mixtures thereof is used.

In step D. the proteolytic system is preferably used at a concentration, with respect to the total biomolecular content in the sample, given as the ratio of enzyme to biomolecular content, in the range of 1/50-1/10000, preferably in the range of 1/100-1/1000 by weight. Step D. can be carried out over a time span of 1-60 minutes, preferably in the range of 2-30 minutes, or 2 - 10 minutes or 2 - 5 minutes, further preferably at a temperature in the range of 20-40°C.

Preferably the temperature in the limited proteolysis step D is in the range of 20-40°C or 4-90°C. The temperature range is normally at around room temperature (20-25°C) or at 37°C; thermolysin on the other hand is active up to 80°C; 4 °C also applicable to slow down proteolytic reaction.

The properties of the used unspecific proteases are summarized in the Table 1 below:

**Table1:**

| *Protease* | *Optimal pH* | *Specificity* |
|---|---|---|
| Proteinase K | 7.5 - 11.0 | Unspecific |
| Thermolysin | 7.0-9.0 | Leu, Phe,Ile, Val, Met, Ala |
| Subtilisin | 7.0 - 11.0 | Unspecific |
| Pepsin | 1.0 - 4.0 | Unspecific |
| Papain | 6.0 - 7.0 | Unspecific |
| α-Chymotrypsin | 7.0 - 9.0 | Phe, Tyr, Trp, Leu, Ile |
| Elastase | 7.5 - 8.8 | Ala, Val, Ile, Leu, Gly, Ser, Thr |

Preferably, in step G. peptides and proteins are removed in a filtration, separation or enrichment step. Preferred methods are including size filtering (for example using a 10 k MWCO filtration device); chromatography including size exclusion, hydrophobic or anion exchange chromatography.

Particularly good results can be obtained if, as preferred, in step G. peptides, proteins or other biomolecules or both having a molar weight larger than 20 kDa, preferably having a molar weight larger than 15 kDa, most preferably having a molar weight larger than 10 kDa are removed from the first fragment sample.

In line with the above good results can also be obtained if, as preferred, in step G. also peptides, having a molar weight smaller than 0.1 kDa, or having a molar weight smaller than 0.2 kDa, or having a molar weight smaller than 0.4 kDa are removed from the first fragment sample.

According to another preferred embodiment, step G. includes, before actual analysis, a proteomics workflow, in particular involving denaturation, C18 cleanup, or a combination thereof.

For quantitative determination heavy labelled fragments characteristic of being the result of the limited proteolysis of step D. as well as remaining after the removal step G., can be spiked into the original complex mixture and/or into the first fragment sample and/or into the enriched fragment sample. So if desired, absolute quantitation can be achieved using heavy-labelled synthetic internal standard peptides. The approach can be directly applied to unfractionated proteome extracts, or it can be coupled to a variety of isotope-labeling and sample fractionation techniques (for example to iTRAQ labeling and the TAILS workflow, O Kleifeld et al., Nature biotechnology 28 (3), 281 (2010)), previously used in proteomic experiments.

For the analytical analysis step, preferably specific, quantitative mass spectrometry-based assays in the form of selected reaction monitoring (SRM) and/or data-independent acquisition of product ion spectra are used.

Also the present invention relates to the use of a method as detailed above in combination with peptide fragment enrichment techniques such as TAILS for the peptides generated by the step D.

The method according to the present invention opens numerous possibilities such as in biomedical, biotechnological and pharmaceutical applications as well as in biological research.

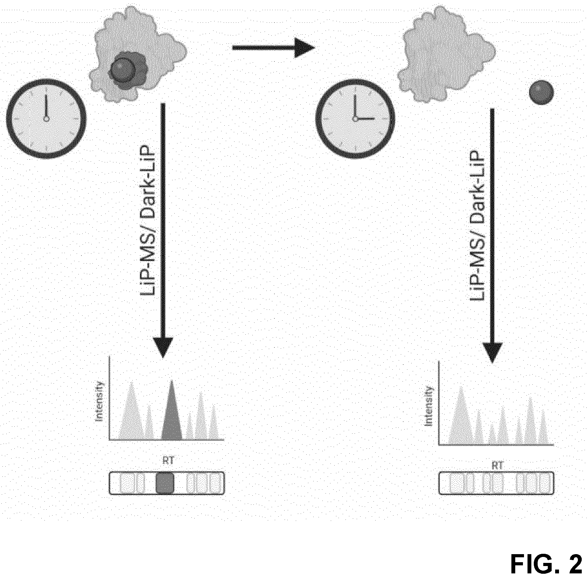

**Fig. 2****.** shows a schematic representation of residence time being measured by LiP-MS/DarkLiP-MS. In this approach, native proteins are incubated with a at least one ligand. The interaction of the ligand with the protein Target induces a structural change in the Target. The ligand is removed from the solution medium, the ligand-bound Targets are incubated for a defined period of time, and then subjected to a limited proteolysis step using LiP-MS or DarkLiP-MS. Finally, the generated peptides are analyzed by mass spectrometry. The peptide abundances measured by mass spectrometry are dependent on the amount of ligand that is still bound to the protein Target at the time of limited proteolysis by LiP-MS or DarkLiP-MS.

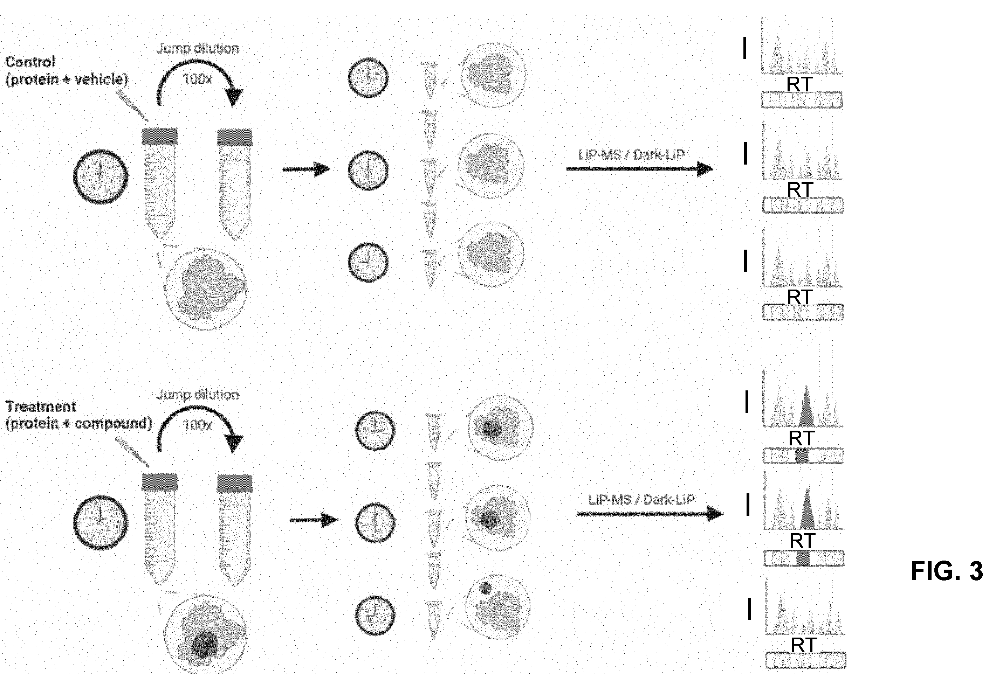

**Fig. 3** shows an experimental workflow used to generate the data shown in the Example section below. Native proteomes were incubated with the ligand of interested (Staurosporine or Calyculin A) - lower panel - or with the solvent (DMSO) - upper panel. The sample treated with DMSO serves as negative control, and it is used to confirm that the observed effect is derived from the interaction of the Ligand with its Targets. The proteomes were then diluted by a factor of 100 (jump-dilution method), and aliquots with a sample of the ligand-bound proteome were extracted at distinct time points after dilution. Due to the reduction of the concentration of the Ligand in solution (as consequence to the jump-dilution), new binding events between Ligand and Target are very unlikely, and consequently it is expected that the proportion of Ligand-bound protein Target decreases over time, as the complex Ligand-Target dissociates. The samples incubated for the distinct times were then subjected to a limited proteolysis step using LiP-MS or DarkLiP-MS, and measured by mass spectrometry. In the right intensity (I) as a function of retention time (RT is given.

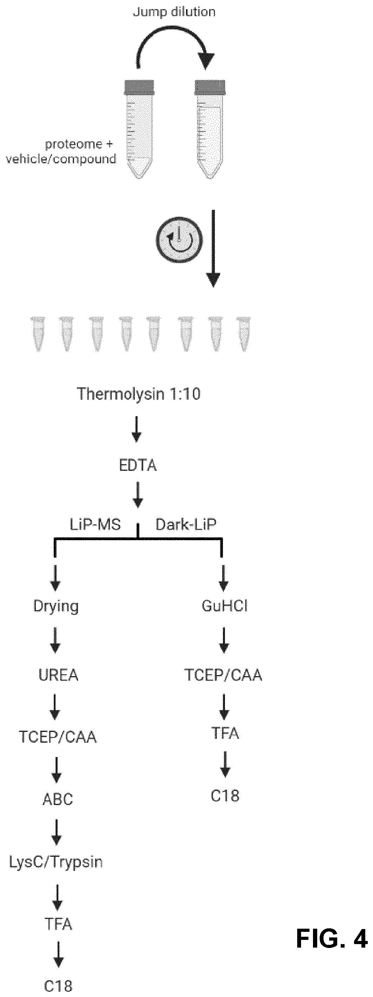

**Fig. 4** shows the experimental workflow used for processing of LiP-MS or DarkLiP-MS samples for analysis by mass spectrometry. For both LiP-MS and DarkLiP-MS, the limited proteolysis is performed with the unspecific protease thermolysin. After incubation for a short amount of time, the reaction is stopped by addition of EDTA.

For processing of LiP-MS experiments (1), the samples are dried after stopping the reaction. After re-solubilization with urea, the partially-fragmented proteome is reduced and alkylated, diluted with ammonium bicarbonate, and treated with LysC and Trypsin to generate a fully fragmented peptide sample. This peptide sample is acidified with TFA and cleaned with a C18 resin before analysis by mass spectrometry.

For processing of DarkLiP-MS experiments (2), the partially-fragmented proteome is denatured with guanidinium-hydrochloride, reduced and alkylated, acidified with TFA, and cleaned with a C18 resin. The direct sample-cleaning with C18, without protein fragmentation with LysC and Trypsin, removes proteins and large peptides still present in solution, and enriches for fragmented peptides generated during the limited proteolysis step. This enrichment step is characteristic of the DarkLiP-MS approach.

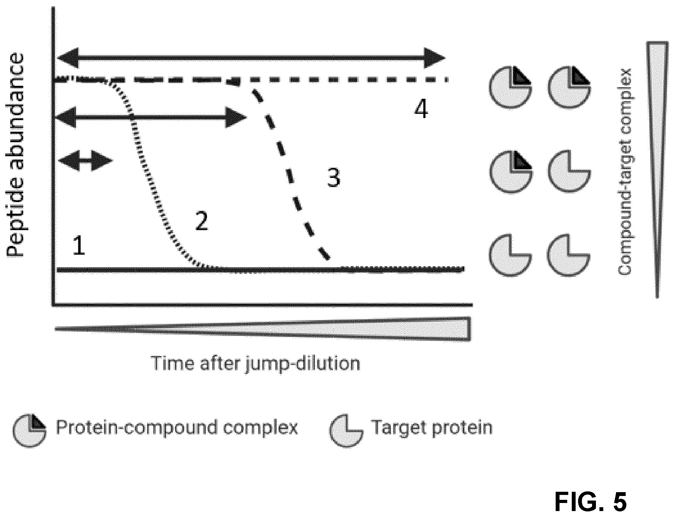

**Fig. 5** shows the expected pattern of peptide abundance recorded for proteins with distinct drug-residence times in comparison with the vehicle. The y-axis of the graph corresponds to the intensity measured for a particular peptide, while the x-axis corresponds to the time after jump-dilution. Since the peptide intensity is dependent on the fraction of Ligand-bound Target protein, the graph shows the life-time of the complex Protein-Ligand.
1- Peptide abundance measured in the vehicle-treated proteome. It is not expected that the Target protein exhibits significant structural changes during experimental procedure. Thus, it is expected that the peptide abundance does not change with the progression of time after dilution of the proteome.
2- Protein with a short drug-residence time. For proteins with short drug-residence times, it is expected that the abundance of the target peptide differs from the abundance of the same peptide measured in the vehicle treated proteome. This is due to the structural change induced by the binding of the Ligand to the Target protein. Since the interaction between Ligand and Target has a short duration, it is expected that the difference in intensity between Ligand-treated and vehicle-treated samples decreases shortly after jump-dilution. Ultimately, the peptide intensity may reach the value of the vehicle-treated sample.
3- Protein with intermediate drug-residence time. For proteins with intermediate drug-residence times, it is also expected that the abundance of the target peptide differs from the abundance of the same peptide measured in the vehicle treated proteome. However, contrarily to the proteins with short drug-residence time described above, the peptide intensity is maintained for a longer period. Ultimately, the peptide intensity may also reach the value of the vehicle-treated sample.
4- Protein with long drug-residence time or irreversible modification. Similarly to the cases 2 and 3, it is also expected that the abundance of the target peptide differs from the abundance of the same peptide measured in the vehicle-treated proteome. However, due to the long or irreversible structural modification, it is expected that the peptide abundance does not change during the measured time period. In this case, the difference of peptide abundance between Ligand-treated proteome and vehicle-treated proteome will be constant.

### Specific Examples

### Example 1: Measurement of residence-time of Staurosporine for the target dual specificity mitogen-activated protein kinase kinase 3 (MP2K3)

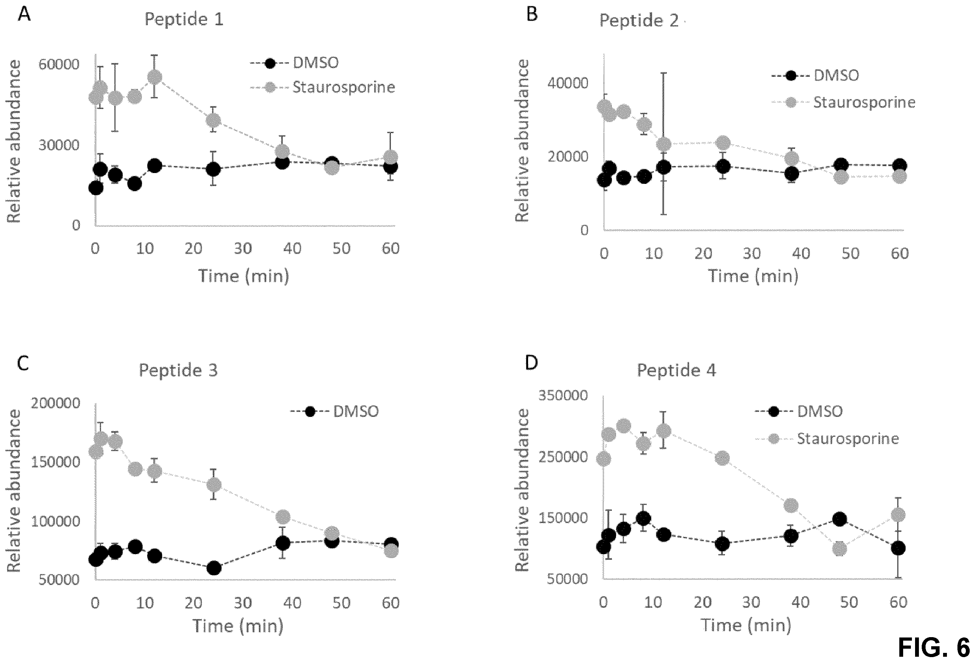

Staurosporine is a natural compound that mimics the biological molecule ATP and acts as a promiscuous kinase inhibitor. Due to the similarities to ATP, Staurosporine has short residence times towards its protein Targets. To show the application of the LiP-MS technology for the measurement of short drug-residence times, the experimental workflow described in **Fig. 3****,** **4** **and** **5** was applied to the promiscuous kinase inhibitor Staurosporine. Native cellular proteomes were treated with Staurosporine or DMSO (vehicle) in duplicates, diluted 100 fold and analyzed by LiP-MS. The results of Example 1 are illustrated in **Fig. 6****.** The graphs of **Fig. 6** show the average abundances (y-axis) of four different peptides Peptide 1 - Peptide 4 of the kinase MP2K3 measured between 0 and 60 minutes after jump-dilution (x-axis). The peptides Peptide 1 - Peptide 4 are described in the database PeptideAtlas with the accessions PAp01457867, Pap00639637, PAp02016489 and PAp00503812. The graphs show a difference in peptide abundance between Staurosporine and DMSO, which is larger at earlier time points after dilution (between 0 and 30 min). The difference in abundance between Staurosporine-treated samples and DMSO decreases has time progresses. This observation shows that the drug Staurosporine bound to its expected target MP2K3, leading to a structural alteration in MP2K3 due to the formation of the complex MP2K3-Staurosporine. The reduction in peptide abundance over time show that the structural alteration disappeared over time (i.e. the complex MP2K3-Staurosporine disassociated), culminating in a structural state like the DMSO treated protein. This result exemplifies the expected pattern for drugs with short residence time, as described in **Fig. 5****.**

Accordingly to equation 1, the residence-time of Staurosporine towards MP2K3 can be determined based on the half-life of the complex MP2K3-Staurosporine. Since the half-life of the complex MP2K3-Staurosporine corresponds to the interval of time that led to a reduction of peptide abundance by 50%, we fit the values of peptide abundance to a 4-parameter logistic curve. Thereby we obtained the time that corresponded to a decrease of relative peptide abundance by 50% (Table 2). Since each peptide generates a slightly different value for the half-life of the complex due experimental variability, we calculated the median of the 4 values obtained based on the four graphs of **Fig. 6****.** Accordingly to equation 1, by dividing the median half-life by 0.693 (the logarithmic value of 2), we obtained a residence time of approximately 65 minutes min for the complex MP2K3-Staurosporine.

**Table 2: Residence time of complex MP2K3-Staurosporine calculated based on four different peptides of MP2K3.**

| Peptide | Half-life (min) | Median half-life (min) | Residence-time (min) |
|---|---|---|---|
| 1 | 25 | | |
| 2 | 61 | 45 | 65 |
| 3 | 129 | | |
| 4 | 29 | | |

### Example 2: Residence-time pattern obtained for the irreversible drug Calyculin A.

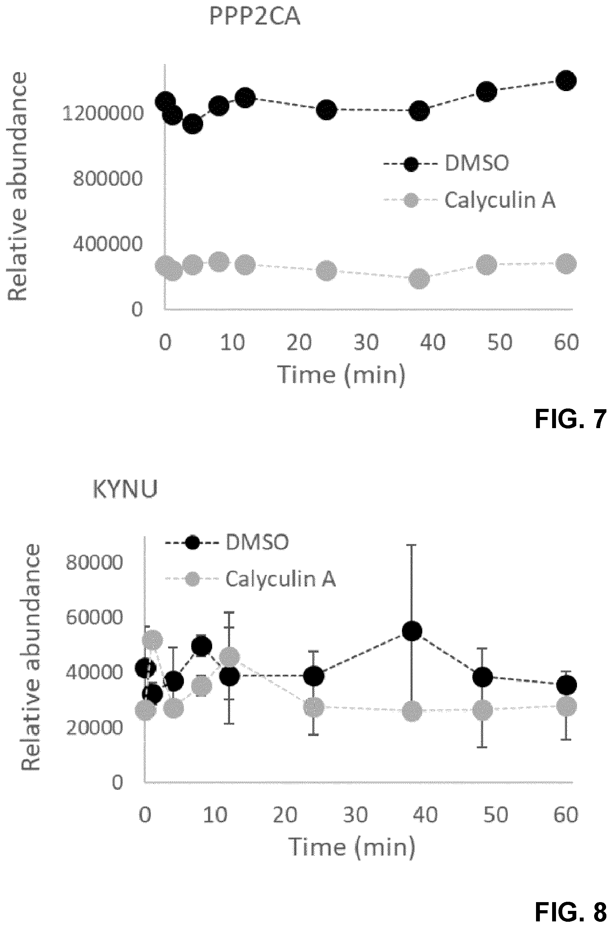

Calyculin A is a natural compound that acts as an potent and irreversible inhibitor of serine/threonine protein phosphatases. To show the application of the LiP-MS technology for the measurement of irreversible drug-residence times, the experimental workflow described in **Fig. 3****,** **4** **and** **5** was applied to the phosphatase inhibitor Calyculin A. Native cellular proteomes were treated with Calyculin A or DMSO (vehicle) in duplicates, diluted 100 fold and analyzed by LiP-MS. The results of Example 2 are illustrated in **Fig. 7 and 8****.** The graph of **Fig. 7** shows the abundance (y-axis) of the Peptide 5 from the protein phoshatase PP2A-alpha (PPP2CA) measured between 0 and 60 minutes after jump-dilution (x-axis). This Peptide is described on the database PeptideAtlas with the accession PAp00524112. The graph shows a constant difference in peptide intensity between Calyculin A and DMSO during the entire length of the experiment. This shows that the drug Calyculin A bound to its expected target PPP2CA, but that the structural alteration derived from the formation of the complex PPP2CA-Calyculin A did not disappear over time. This result exemplifies the expected pattern for irreversible drugs, as described in **Fig. 5****.**

The graph of **Fig. 8** shows the abundance (y-axis) of the Peptide 6 from the protein Kynureninase (KYNU), a Non-Target of Calyculin A. This Peptide is described on the database PeptideAtlas with the accession PAp04420703. The graph shows that Calyculin A did not bind to KYNU and consequently did not induce structural alterations in KYNU, since the peptide intensity is similar in Calyculin A and DMSO treated samples during the entire length of the experiment. The divergence between the lines of Calyculin A and DMSO can be explained by experimental variability, since the scale on the y-axis represents a much smaller variation than in the graphs of **Fig. 6** **and** **7** (where real structural changes are expected).

### LIST OF REFERENCE SIGNS

| | | | |
|---|---|---|---|
| 100 | native proteins | 103 | filtration |
| 101a | ligand | 104a/b | unique peptide populations |
| 101b | control (vehicle) samples | I | intensity |
| 102 | limited digest step | RT | retention time |

## Claims

1. A method to determine the residence time between at least one Target and at least one Ligand, said Target contained in a complex cellular mixture of at least one of further Targets and other biomolecules, comprising the following steps:
- Step A: Incubating said at least one Target with said at least one Ligand, for incubation period of time with a binding reaction between said Ligand and said Target,
- Step B: Stopping said binding reaction by removing said at least one Ligand in as far as not bound to Target; or reducing the number of binding events by reducing the concentration of the Ligand,
- Step C: Incubating said at least one Ligand bound to said at least one Target for different periods of time, leading to an incubated Ligand bound mixture,
- Step D: Limited proteolysis of the incubated Ligand bound mixture under a condition in which said at least one Target is in the original conformational state to be detected leading to a first fragment sample,
wherein this step D is followed by either the sequence of:
- Step E: Denaturation of the sample to a denaturated first fragment sample,
- Step F. Digestion of the sample to obtain a completely fragmented sample,
or this Step D is followed by the following step:
- Step G: Removal of large peptides and proteins or other biomolecules from said first fragment sample to form an enriched fragment sample,
for both cases followed by the steps:
- an analytical analysis, wherein quantitative mass spectrometry-based assays in the form of at least one of selected/parallel reaction monitoring (SRM/PRM) and data-independent acquisition of product ion spectra is used,
∘ wherein for the case of Steps E and F, the analytical analysis is run on the completely fragmented sample for the determination of fragments characteristic being the result of both the limited proteolysis step D. as well as of the complete fragmentation step F,
∘ and wherein for the case of Step G the analytical analysis is run on the enriched fragment sample,
- measurement of peptide abundance over distinct incubation times post Ligand removal, to determine the dissociation half-life,
the residence time (τ) being calculated therefrom according to the following equation $τ = \frac{1}{koff} = \frac{t 1 / 2}{ln 2} = \frac{t 1 / 2}{0.693}$ with
*τ* = Residence time
k_{off} = dissociation constant
t_{1/2} = Dissociation half-life
wherein for the case of Steps E and F, the determination of the residence time between the Target and the Ligand is based on a quantitative comparison of the analytical analysis of the completely fragmented sample with the analytical analysis of the completely fragmented control sample
and wherein for the case of Step G the determination of the residence time between the Target and the Ligand is based on a quantitative comparison of the analytical analysis of the enriched fragment sample with the analytical analysis of the enriched fragmented control sample.

2. Method according to claim 1, wherein the Limited proteolysis (step D) of the complex mixture is carried out over a time span of 1-60 minutes, preferably in the range of 2-30 minutes, or 2 - 10 minutes or 2 - 5 minutes, preferably at a temperature in the range of 20-40°C.

3. Method according to claim 2, wherein for the case of Steps E and F in the digestion step F Trypsin is used, preferably at a temperature of 15 -70°C, over a time span of 2 to 24 hours, at an enzyme to substrate weight ratio in the range of 1/10.

4. A method according to any of claims 1 to 3 wherein the Target is a protein or at least a portion of a proteome under native condition and wherein the Ligand comprises one of the following entities: a small drug compound, a natural occurring protein cofactor, coenzyme or co-substrate, including a metal ion or an organic molecule, an amino acid, a synthetic peptide-drug, a natural occurring peptide, a macromolecule including another protein, nucleic acid, lipid or macromolecular complex.

5. A method according to any of claims 1 to 4 wherein the at least one Target is a protein based exclusively on proteinogenic amino acids or is based on proteinogenic amino acids and carries post-translational modifications and wherein the complex mixture of further proteins and/or other biomolecules is a complex native biological matrix.

6. Method according to any of the preceding claims, wherein in the limited proteolysis step D the proteolytic system is selected from the group consisting of proteinase K, Thermolysin, Subtilisin, Pepsin, Papain, α-Chymotrypsin, Elastase, and mixtures thereof is used.

7. Method according to any of the preceding claims, wherein in the Limited proteolysis step D the proteolytic system is used at a concentration, with respect to the total biomolecular content in the sample, given as the ratio of enzyme to biomolecular content, in the range of 1/25-1/10000, preferably in the range of 1/50-1/1000 by weight.

8. Method according to any of the preceding claims, wherein if DarkLiP-MS protocol is used the removal ( step G) of large peptides and proteins is done in a filtration, separation or enrichment step, including size filtering; chromatography including size exclusion, hydrophobic or anion exchange chromatography; physical removal including phase separation, absorption, precipitation; filtration, separation or enrichment based on hydrophilic/hydrophobic properties; filtration, separation or enrichment based on electric/magnetic field; or a combination thereof.

9. Method according to any of the preceding claims, wherein for the case of Step G peptides, proteins and/or other biomolecules having a molar weight larger than 20 kDa, preferably having a molar weight larger than 15 kDa, most preferably having a molar weight larger than 10 kDa are removed (step G) from the first fragment sample.

10. Method according to any of the preceding claims, wherein before the analytical analysis step, a proteomics workflow, in particular involving denaturation, C18 cleanup, or a combination thereof is carried.

11. Method according to any of the preceding claims, where for the analytical analysis SWATH-MS and/or optionally data-dependent acquisition (shotgun), is used.

12. Method according to any of the preceding claims, wherein for quantitative mass spectrometry-based assays heavy labelled fragments characteristic of being the result of the limited proteolysis or of the complete fragmentation are spiked into the original complex mixture or into the completely fragmented sample.

13. Method according to any of the preceding claims, wherein for quantitative mass spectrometry-based assays heavy labelled fragments characteristic of being the result of the limited proteolysis of Step D. as well as remaining after the removal of large peptides and proteins or other biomolecules of Step G, are spiked into the original complex mixture and/or into the first fragment sample and/or into the enriched fragment sample.

14. Use of the method according to any of the preceding claims for at least one of the following purposes
- to prioritize Target candidates based on their residence time
- to select best drug-target candidates,
- to determine medically relevant Target,
- to predict the in-vivo off target/ side effect of different drugs based on their binding kinetics,
- to control the quality of protein-based pharmaceutical preparations.

15. A method aiding in the prognostic or diagnosis of disorders pathologies in patients early in the progression of the dysfunction/disease comprising determining the presence of at least one selected target protein and determining the residence time between at least said selected target protein and at least one ligand according to any of claims 1 to Claim 14 in a patient's biologic sample such as plasma, serum, cerebrospinal fluid or urine and comparing the concentration data to concentration data from populations affected by this disorder/ disease to verify or nullify the presence of the given pathologies.

## Patentansprüche

1. Verfahren zum Bestimmen der Verweilzeit zwischen mindestens einem Ziel und mindestens einem Liganden, wobei das Ziel in einer komplexen zellulären Mischung von mindestens einem weiteren Ziel und anderen Biomolekülen enthalten ist, umfassend die folgenden Schritte:
- Schritt A: Inkubieren des mindestens einen Ziels mit dem mindestens einen Liganden für einen Inkubationszeitraum mit einer Bindungsreaktion zwischen dem Liganden und dem Ziel,
- Schritt B: Anhalten der Bindungsreaktion durch Entfernen des mindestens einen Liganden, soweit dieser nicht an das Ziel gebunden ist; oder Reduzieren der Anzahl der Bindungsereignisse durch Reduzieren der Konzentration des Liganden,
- Schritt C: Inkubieren des mindestens einen an das mindestens eine Ziel gebundenen Liganden über unterschiedliche Zeiträume, führend zu einer inkubierten, ligandengebundenen Mischung,
- Schritt D: Begrenzte Proteolyse der inkubierten, ligandengebundenen Mischung unter einer Bedingung, bei der sich das mindestens eine Ziel in dem ursprünglichen, zu erkennenden Konformationszustand befindet, führend zu einer ersten Fragmentprobe,
wobei dieser Schritt D gefolgt wird von entweder der Sequenz:
- Schritt E: Denaturierung der Probe zu einer denaturierten ersten Fragmentprobe,
- Schritt F: Aufschluss der Probe, um eine vollständig fragmentierte Probe zu erhalten,
oder dieser Schritt D wird von dem folgenden Schritt gefolgt:
- Schritt G: Entfernung großer Peptide und Proteine oder anderer Biomoleküle aus der ersten Fragmentprobe, um eine angereicherte Fragmentprobe zu bilden,
für beide Fälle folgen die Schritte:
- einer analytischen Analyse, wobei quantitative massenspektrometriebasierte Untersuchungen in Form von mindestens einem der folgenden verwendet werden: Selected Reaction Monitoring (SRM) bzw. Parallel Reaction Monitoring (PRM) und die datenunabhängige Erfassung von Produktionenspektren,
∘ wobei bei den Schritten E und F die analytische Analyse an der vollständig fragmentierten Probe zur Bestimmung von Fragmenten läuft, die das Ergebnis sowohl des begrenzten Proteolyse-Schritts D als auch des vollständigen Fragmentierungsschritts F sind,
o und wobei bei Schritt G die analytische Analyse an der angereicherten Fragmentprobe läuft,
- Messung des Peptidüberflusses über verschiedene Inkubationszeiten nach der Ligandenentfernung, um die Dissoziationshalbwertszeit zu bestimmen,
wobei die Verweilzeit (τ) daraus gemäß der folgenden Gleichung berechnet wird $τ = \frac{1}{koff} = \frac{t 1 / 2}{ln 2} = \frac{t 1 / 2}{0.693}$ mit
τ = Verweilzeit
k_{off} = Dissoziationskonstante
t_{1/2} = Dissoziationshalbwertszeit
wobei bei den Schritten E und F die Bestimmung der Verweilzeit zwischen dem Ziel und dem Liganden auf einem quantitativen Vergleich der analytischen Analyse der vollständig fragmentierten Probe mit der analytischen Analyse der vollständig fragmentierten Kontrollprobe basiert
und wobei bei Schritt G die Bestimmung der Verweilzeit zwischen dem Ziel und dem Liganden auf einem quantitativen Vergleich der analytischen Analyse der angereicherten Fragmentprobe mit der analytischen Analyse der angereicherten fragmentierten Kontrollprobe basiert.

2. Verfahren nach Anspruch 1, wobei die begrenzte Proteolyse (Schritt D) der komplexen Mischung über einen Zeitraum von 1 bis 60 Minuten, vorzugsweise im Bereich von 2 bis 30 Minuten, oder von 2 bis 10 Minuten oder 2 bis 5 Minuten, vorzugsweise bei einer Temperatur im Bereich von 20 bis 40 °C, durchgeführt wird.

3. Verfahren nach Anspruch 2, wobei bei den Schritten E und F im Aufschlussschritt F Trypsin verwendet wird, vorzugsweise bei einer Temperatur von 15 bis 70 °C, über einen Zeitraum von 2 bis 24 Stunden bei einem Gewichtsverhältnis von Enzym zu Substrat im Bereich von 1/10.

4. Verfahren nach einem der Ansprüche 1 bis 3, wobei das Ziel ein Protein oder mindestens ein Teil eines Proteoms unter nativen Bedingungen ist und wobei der Ligand eine der folgenden Einheiten umfasst: eine kleine Wirkstoffverbindung, einen natürlich vorkommenden Proteinkofaktor, ein natürlich vorkommendes Coenzym oder ein natürlich vorkommendes Co-Substrat, einschließlich eines Metallions oder eines organischen Moleküls, eine Aminosäure, einen synthetischen Peptidwirkstoff, ein natürlich vorkommendes Peptid, ein Makromolekül einschließlich eines anderen Proteins, einer anderen Nukleinsäure, eines anderen Lipids oder eines makromolekularen Komplexes.

5. Verfahren nach einem der Ansprüche 1 bis 4, wobei das mindestens eine Ziel ein Protein ist, das ausschließlich auf proteinogenen Aminosäuren basiert, oder auf proteinogenen Aminosäuren basiert und posttranslationale Modifikationen trägt, und wobei die komplexe Mischung von weiteren Proteinen und/oder anderen Biomolekülen eine komplexe native biologische Matrix ist.

6. Verfahren nach einem der vorhergehenden Ansprüche, wobei in dem begrenzten Proteolyseschritt D das proteolytische System ausgewählt ist aus der Gruppe, bestehend aus Proteinase K, Thermolysin, Subtilisin, Pepsin, Papain, α-Chymotrypsin, Elastase, und Mischungen davon verwendet werden.

7. Verfahren nach einem der vorhergehenden Ansprüche, wobei im begrenzten Proteolyseschritt D das proteolytische System in einer Konzentration verwendet wird, die, bezüglich des gesamten biomolekularen Gehalt in der Probe, als Verhältnis von Enzym zu biomolekularem Gehalt im Bereich von 1/25 bis 1/10.000, vorzugsweise im Bereich von 1/50 bis 1/1.000 bezogen auf das Gewicht, gegeben ist.

8. Verfahren nach einem der vorhergehenden Ansprüche, wobei bei Verwendung des DarkLiP-MS-Protokolls die Entfernung (Schritt G) großer Peptide und Proteine in einem Filtrations-, Trenn- oder Anreicherungsschritt erfolgt, einschließlich Größenfiltration; Chromatographie, einschließlich Größenausschluss-, hydrophober oder Anionenaustauschchromatographie; physikalische Entfernung, einschließlich Phasentrennung, Absorption, Ausfällung; Filtration, Trennung oder Anreicherung basierend auf hydrophilen/hydrophoben Eigenschaften; Filtration, Trennung oder Anreicherung basierend auf elektrischen/magnetischen Feldern; oder eine Kombination davon.

9. Verfahren nach einem der vorhergehenden Ansprüche, wobei bei Schritt G Peptide, Proteine und/oder andere Biomoleküle mit einem Molekulargewicht von größer als 20 kDa, vorzugsweise mit einem Molekulargewicht von größer als 15 kDa, am meisten bevorzugt mit einem Molekulargewicht von größer als 10 kDa, aus der ersten Fragmentprobe entfernt werden (Schritt G).

10. Verfahren nach einem der vorhergehenden Ansprüche, wobei vor dem analytischen Analyseschritt ein Proteomik-Arbeitsablauf durchgeführt wird, der insbesondere eine Denaturierung, eine C18-Reinigung oder eine Kombination davon beinhaltet.

11. Verfahren nach einem der vorhergehenden Ansprüche, wobei für die analytische Analyse SWATH-MS und/oder optional datenabhängige Erfassung (Shotgun) verwendet wird.

12. Verfahren nach einem der vorhergehenden Ansprüche, wobei für quantitative massenspektrometriebasierte Untersuchungen schwere markierte Fragmente, die charakteristisch für das Ergebnis der begrenzten Proteolyse oder der vollständigen Fragmentierung sind, in die ursprüngliche komplexe Mischung oder in die vollständig fragmentierte Probe dotiert werden.

13. Verfahren nach einem der vorhergehenden Ansprüche, wobei für quantitative massenspektrometriebasierte Untersuchungen schwere markierte Fragmente, die charakteristisch für das Ergebnis der begrenzten Proteolyse aus Schritt D sind sowie nach der Entfernung großer Peptide und Proteine oder anderer Biomoleküle aus Schritt G verbleiben, in die ursprüngliche komplexe Mischung und/oder in die erste Fragmentprobe und/oder in die angereicherte Fragmentprobe dotiert werden.

14. Verwendung des Verfahrens nach einem der vorhergehenden Ansprüche für mindestens einen der folgenden Zwecke
- zum Priorisieren von Zielkandidaten basierend auf ihrer Verweilzeit
- zum Auswählen der besten Wirkstoff-Ziel-Kandidaten,
- zum Bestimmen des medizinisch relevanten Ziels,
- zum Vorhersagen der In-vivo-Off-Target-/Nebenwirkungen von unterschiedlichen Wirkstoffen basierend auf deren Bindungskinetik,
- zur Qualitätskontrolle von proteinbasierten pharmazeutischen Zubereitungen.

15. Verfahren zum Unterstützen der Prognose oder Diagnose von Störungspathologien bei Patienten in einem frühen Stadium des Fortschreitens der Dysfunktion/Krankheit, umfassend das Bestimmen des Vorhandenseins mindestens eines ausgewählten Zielproteins und das Bestimmen der Verweilzeit zwischen mindestens dem ausgewählten Zielprotein und mindestens einem Liganden nach einem der Ansprüche 1 bis 14 in der biologischen Probe eines Patienten, wie zum Beispiel Plasma, Serum, Liquor oder Urin, und Vergleichen der Konzentrationsdaten mit Konzentrationsdaten von Populationen, die von dieser Krankheit/Störung betroffen sind, um das Vorhandensein der gegebenen Pathologien zu bestätigen oder aufzuheben.

## Revendications

1. Procédé pour déterminer le temps de résidence entre au moins une Cible et au moins un Ligand, ladite Cible étant contenue dans un mélange cellulaire complexe d'au moins l'une de Cibles supplémentaires et autres biomolécules, comprenant les étapes suivantes :
- étape A : incubation de ladite au moins une Cible avec ledit au moins un Ligand pendant une période de temps d'incubation avec une réaction de liaison entre ledit Ligand et ladite Cible,
- étape B : interruption de ladite réaction de liaison par élimination dudit au moins un Ligand dans la mesure où il n'est pas lié à la Cible ; ou réduction du nombre d'événements de liaison par réduction de la concentration du Ligand,
- étape C : incubation dudit au moins un Ligand lié à ladite au moins une Cible pour différentes périodes de temps, conduisant à un mélange lié incubé de Ligand,
- étape D : protéolyse limitée du mélange de Ligand lié incubé dans une condition dans laquelle ladite au moins une Cible est dans l'état conformationnel d'origine à détecter conduisant à un premier échantillon de fragments,
dans lequel cette étape D est suivie par la séquence suivante :
- étape E : dénaturation de l'échantillon en un premier échantillon de fragments dénaturé,
- étape F : digestion de l'échantillon pour obtenir un échantillon complètement fragmenté,
ou bien cette étape D est suivie par l'étape suivante :
- étape G : élimination des peptides et protéines de grande taille ou d'autres biomolécules dudit premier échantillon de fragments pour former un échantillon de fragments enrichi,
dans les deux cas suivie par les étapes suivantes :
- une analyse analytique, dans laquelle des dosages quantitatifs basés sur la spectrométrie de masse sont utilisés sous la forme d'au moins l'une de la surveillance de réaction sélectionnée/parallèle (SRM/PRM) et de l'acquisition de spectres d'ions produits indépendante des données,
∘ dans lequel dans le cas des étapes E et F, l'analyse analytique est effectuée sur l'échantillon complètement fragmenté pour la détermination de caractéristique de fragments étant le résultat d'à la fois l'étape D de protéolyse limitée ainsi que de l'étape F de fragmentation complète,
∘ et dans lequel dans le cas de l'étape G, l'analyse analytique est effectuée sur l'échantillon de fragments enrichi,
- une mesure d'abondance des peptides sur des temps d'incubation distincts après l'élimination du ligand, pour déterminer la demi-vie de dissociation,
le temps de résidence (τ) étant calculé à partir de celle-ci selon l'équation suivante $τ = \frac{1}{koff} = \frac{t 1 / 2}{ln 2} = \frac{t 1 / 2}{0.693}$ avec
τ = temps de résidence
k_{off} = constante de dissociation
t_{1/2} = demi-vie de dissociation
dans lequel dans le cas des étapes E et F, la détermination du temps de résidence entre la Cible et le Ligand est basée sur une comparaison quantitative de l'analyse analytique de l'échantillon complètement fragmenté avec l'analyse analytique de l'échantillon témoin complètement fragmenté,
et dans lequel dans le cas de l'étape G, la détermination du temps de résidence entre la Cible et le Ligand est basée sur une comparaison quantitative de l'analyse analytique de l'échantillon de fragments enrichi avec l'analyse analytique de l'échantillon témoin fragmenté enrichi.

2. Procédé selon la revendication 1, dans lequel la protéolyse limitée (étape D) du mélange complexe est réalisée sur un intervalle de temps de 1 à 60 minutes, de préférence dans la plage de 2 à 30 minutes ou de 2 à 10 minutes ou de 2 à 5 minutes, de préférence à une température dans la plage de 20 à 40 °C.

3. Procédé selon la revendication 2, dans lequel dans le cas des étapes E et F, dans l'étape de digestion F de la Trypsine est utilisée, de préférence à une température de 15 à 70 °C, sur un intervalle de temps de 2 à 24 heures, à un rapport de poids entre l'enzyme et le substrat dans la plage de 1/10.

4. Procédé selon l'une quelconque des revendications 1 à 3, dans lequel la Cible est une protéine ou au moins une partie d'un protéome dans une condition native et dans lequel le ligand comprend l'une des entités suivantes : un petit composé médicamenteux, un cofacteur, coenzyme ou co-substrat protéique d'origine naturelle, incluant un ion métallique ou une molécule organique, un acide aminé, un peptide-médicament de synthèse, un peptide d'origine naturelle, une macromolécule incluant une autre protéine, un autre acide nucléique, lipide ou complexe macromoléculaire.

5. Procédé selon l'une quelconque des revendications 1 à 4, dans lequel l'au moins une Cible est une protéine basée exclusivement sur des acides aminés protéinogènes ou est basée sur des acides aminés protéinogènes et porte des modifications post-traductionnelles et dans lequel le mélange complexe de protéines supplémentaires et/ou d'autres biomolécules est une matrice biologique native complexe.

6. Procédé selon l'une quelconque des revendications précédentes, dans lequel dans l'étape D de protéolyse limitée, le système protéolytique est sélectionné dans le groupe consistant en protéinase K, thermolysine, subtilisine, pepsine, papaïne, α-chymotrypsine, élastase et mélange de ceux-ci.

7. Procédé selon l'une quelconque des revendications précédentes, dans lequel dans l'étape D de protéolyse limitée, le système protéolytique est utilisé à une concentration, par rapport au contenu biomoléculaire total dans l'échantillon, exprimée comme le rapport entre l'enzyme et le contenu biomoléculaire, dans la plage de 1/25 à 1/10000, de préférence dans la plage de 1/50 à 1/1000 en poids.

8. Procédé selon l'une quelconque des revendications précédentes, dans lequel si le protocole DarkLip-MS est utilisé, l'élimination (étape G) des peptides et protéines de grande taille est effectuée dans une étape de filtration, de séparation ou d'enrichissement, incluant le filtrage par taille ; la chromatographie incluant l'exclusion par taille, la chromatographie hydrophobe ou d'échange d'anions ; l'élimination physique incluant la séparation des phases, l'absorption, la précipitation ; la filtration, la séparation ou l'enrichissement basés sur des propriétés hydrophiles/hydrophobes ; la filtration, la séparation ou l'enrichissement basés sur le champ électrique/magnétique ; ou une combinaison de ceux-ci.

9. Procédé selon l'une quelconque des revendications précédentes, dans lequel dans le cas de l'étape G, les peptides, protéines et/ou autres biomolécules ayant un poids molaire supérieur à 20 kDa, de préférence ayant un poids molaire supérieur à 15 kDa, plus préférablement ayant un poids molaire supérieur à 10 kDa sont éliminés (étape G) du premier échantillon de fragments.

10. Procédé selon l'une quelconque des revendications précédentes, dans lequel avant l'étape d'analyse analytique, un flux de travail de protéomique, impliquant en particulier la dénaturation, la purification sur C18 ou une combinaison de ceux-ci est réalisé.

11. Procédé selon l'une quelconque des revendications précédentes, où pour l'analyse analytique une SWATH-MS et/ou éventuellement une acquisition dépendante des données (shotgun), est utilisée.

12. Procédé selon l'une quelconque des revendications précédentes, dans lequel pour des dosages quantitatifs basés sur la spectrométrie de masse, des fragments marqués lourds, ayant pour caractéristique d'être le résultat de la protéolyse limitée ou de la fragmentation complète, sont ajoutés dans le mélange complexe d'origine ou dans l'échantillon complètement fragmenté.

13. Procédé selon l'une quelconque des revendications précédentes, dans lequel pour des dosages quantitatifs basés sur la spectrométrie de masse, des fragments marqués lourds, ayant pour caractéristique d'être le résultat de la protéolyse limitée de l'étape D ainsi que de subsister après l'élimination des peptides et de protéines de grande taille ou d'autres biomolécules de l'étape G, sont ajoutés dans le mélange complexe d'origine et/ou dans le premier échantillon de fragments et/ou dans l'échantillon de fragments enrichi.

14. Utilisation du procédé selon l'une quelconque des revendications précédentes pour au moins l'un des objectifs suivants
- prioriser les candidats Cible sur la base de leur temps de résidence
- sélectionner les meilleurs candidats médicament-cible,
- déterminer la Cible pertinente sur le plan médical,
- prédire les effets hors cible/secondaires in-vivo de différents médicaments sur la base de leur cinétique de liaison,
- contrôler la qualité des préparations pharmaceutiques à base de protéines.

15. Procédé d'aide dans le pronostic ou le diagnostic de troubles et pathologies chez des patients à un stade précoce de la progression de la dysfonction/maladie comprenant la détermination de la présence d'au moins une protéine cible sélectionnée et la détermination du temps de résidence entre au moins ladite protéine cible sélectionnée et au moins un ligand selon l'une quelconque des revendications 1 à 14 dans un échantillon biologique du patient tel que plasma, sérum, fluide cérébrospinal ou urine et la comparaison des données de concentration à des données de concentration provenant de populations affectées par ce trouble/cette maladie pour vérifier ou neutraliser la présence des pathologies données.
